(19) Europäisches Patentamt

European Patent Office

Office européen des brevets

(11) **EP 1 728 515 A1**

(12) **EUROPEAN PATENT APPLICATION**
published in accordance with Art. 158(3) EPC

(43) Date of publication:
**06.12.2006 Bulletin 2006/49**

(51) Int Cl.:
*A61K 38/00* (2006.01)    *A61P 31/16* (2006.01)
*A61P 43/00* (2006.01)

(21) Application number: **04718765.3**

(22) Date of filing: **09.03.2004**

(86) International application number:
**PCT/JP2004/003031**

(87) International publication number:
**WO 2005/084694 (15.09.2005 Gazette 2005/37)**

(84) Designated Contracting States:
**AT BE BG CH CY CZ DE DK EE ES FI FR GB GR HU IE IT LI LU MC NL PL PT RO SE SI SK TR**

(71) Applicant: **GLYCOMEDICS, INC.**
**Tokyo**
**105-0001 (JP)**

(72) Inventor: **SATO, Toshinori,**
**c/o Keio University**
**Yokohama-shi, Kanagawa 2238522 (JP)**

(74) Representative: **Jones, Helen M.M.**
**Gill Jennings & Every LLP**
**Broadgate House**
**7 Eldon Street**
**London EC2M 7LH (GB)**

(54) **INFLUENZA VIRUS-INFECTION INHIBITOR**

(57)    The present invention is directed to providing influenza virus infection suppressors which contain a sialylgalactose-binding peptide, for example, a ganglioside GM3-binding peptide, as an active ingredient. Specifically, the invention provides influenza virus infection suppressors which contain the sialylgalactose-binding peptide of the following (a) or (b): (a) a peptide having the amino acid sequence of SEQ ID NO. 1 or 2; (b) a peptide having an amino acid sequence in which one or several nucleotides are deleted, substituted, or added, from/to the amino acid sequence of SEQ ID NO. 1 or 2, and having sialylgalactose-binding activity.

EP 1 728 515 A1

SEQUENCE LISTING

<110> GLYCOMEDICS

<120> INFLUENZA VIRUS INFECTION SUPPRESSOR

<130> HMJ04273EP

<160> 8

<170> PatentIn version 3.1

<210> 1
<211> 15
<212> PRT
<213> Artificial Sequence

<220>
<223> Description of Artificial Sequence:Synthetic peptide

<400> 1

Gly Trp Trp Tyr Lys Gly Arg Ala Arg Pro Val Ser Ala Val Ala
1               5                   10                  15


<210> 2
<211> 15
<212> PRT
<213> Artificial Sequence

<220>
<223> Description of Artificial Sequence:Synthetic peptide

<400> 2

Arg Ala Val Trp Arg His Ser Val Ala Thr Pro Ser His Ser Val
1               5                   10                  15


<210> 3
<211> 15
<212> PRT
<213> Artificial Sequence

<220>
<223> Description of Artificial Sequence:Synthetic peptide

<400> 3

Leu Ser Trp Pro Leu His Ala Gly Arg Gly Phe Arg Trp Val Ser
1               5                   10                  15


<210> 4
<211> 15
<212> PRT
<213> Artificial Sequence

<220>
<223> Description of Artificial Sequence:Synthetic peptide

<400> 4

```
Gly Trp Tyr Ser Ser Arg His Tyr Val Arg Ser Leu Asn Gly Leu
1               5               10                  15
```

```
<210>   5
<211>   15
<212>   PRT
<213>   Artificial Sequence

<220>
<223>   Description of Artificial Sequence:Synthetic peptide

<400>   5
```

```
Gln Gln Leu Val Tyr Asn Trp Trp Ala Val Ser Ser Ala Arg Arg
1               5               10                  15
```

```
<210>   6
<211>   15
<212>   PRT
<213>   Artificial Sequence

<220>
<223>   Description of Artificial Sequence:Synthetic peptide

<400>   6
```

```
Leu Trp Arg Pro Val Leu Phe His Ser Ala Val Arg Ala Leu Gly
1               5               10                  15
```

```
<210>   7
<211>   15
<212>   PRT
<213>   Artificial Sequence

<220>
<223>   Description of Artificial Sequence:Synthetic peptide

<400>   7
```

```
Trp Arg Gly Val Tyr Phe Gly Asp Arg Trp Leu Gly Ser Gln Pro
1               5               10                  15
```

```
<210>   8
<211>   15
<212>   PRT
<213>   Artificial Sequence

<220>
<223>   Description of Artificial Sequence:Synthetic peptide

<400>   8
```

```
Ala Arg Leu Ser Pro Thr Met Val His Pro Asn Gly Ala Gln Pro
1               5               10                  15
```

**Description**

TECHNICAL FIELD

The present invention relates to influenza virus infection suppressors which contain a sialylgalactose-binding peptide, for example, a ganglioside GM3-binding peptide, as an active ingredient.

BACKGROUND ART

The oligosaccharides of glycoconjugates, such as glycolipids, glycoproteins, and proteoglycans on the cell surface, are target molecules for recognition by extracellular molecules. Such oligosaccharides are associated with cell-specific hinging process (references (1), (2)). The sialylgalactose (NeuAc-Gal) structure is well known as a receptor for bacterial toxins, viruses, endothelial cells, etc. (references (3)-(5)1. Sialic acid-containing glycosphingolipids, so-called gangliosides, are associated with important biological functions which are dependent on their carbohydrate portion (references (6)-(8)). In the past decade, studies on localization and distribution of membrane components, such as lipids, glycolipids, and other components, have focused on the relationship between their characteristics and functions (references (9)-(12)). Most glycosphingolipids were extracted from cells as detergent-insoluble membranes together with signal transduction molecules (references (13)-(15)). It has been found that the glycolipid-enriched subtraction is modified by cell stimulation and signaling. The membrane containing glycolipids were reconstructed as an air-water interface monolayer (references (16) - (18)). Sato et al. reported that the lectin binding pattern is dependent on distribution of glycosphingolipids in a lipid monolayer (references (19)-(22)). Information on the structure and function of a glycolipid-enriched microdomain in a membrane will facilitate clarification of the biological role of sugar chains on the cell surface. A number of sugar-binding proteins such as lectins and antibodies have been used for labeling glycoconjugates, leading to applications in the therapy of carbohydrate-related diseases (references (23), (24)).

The biological selection system (phage display library) was developed to select peptides which specifically bind to target molecules (reference (25), (26)). Use of this system has facilitated many selections of various glycoconjugates, monosaccharides (reference (27), (28)), the tumor-associated glycoantigen (reference (29)-(32)). sialyl Lewis[x] (reference (33), (34)), glycosphingolipids (reference (35), (36)), proteoglycans (reference (37)-(39)) and polysaccharides (references (40), (41)). The inventors of the present application performed selection of ganglioside GM1 (Galβ1 → 3GalNAcβ1 → 4 (NeuAcα2 → 3) Galβ1 → 4Glcβ1 → 1' Cer) binding peptides in a previous paper (reference (35)). It was shown that the selected peptide sequences bound specifically to GM1, thereby inhibiting the binding of the cholera toxin to GM1.

Influenza viruses have two kinds of glycoproteins in their envelope membranes: hemagglutinin (HA) and sialidase (neuraminidase), each of which plays an important role in an establishment of viral infection and a viral budding from a host cell, respectively. Hemagglutinin recognizes a sialic acid-containing sugar chain present on the cell membrane of an animal host as a receptor, and specifically binds to such a receptor, leading to intracellular endocytosis of the influenza virus. Sialidase, a receptor-destroying enzyme, cleaves a sialic acid residue on the host cell membrane or virus' own membrane when the viral particle buds or is released from the host cell.

Currently, there are few treatment options with preventive/therapeutic agents against influenza virus infection. Vaccines are used to prevent infection, but only in a limited range because an injection is required. As for a therapeutic agent, the inhibitor of sialidase, a membrane protein of an influenza virus, has been recently used in the clinical situation. The sialidase inhibitor is considered to suppress extracellular release of influenza viruses after their intracellular propagation. Therefore, the sialidase inhibitor is not effective as a preventive agent. Moreover, its application has been limited because it should be administered as soon after the infection as one or two days.

Based on the finding that, during the infection process of an influenza virus, hemagglutinin binds to a sialic acid-containing sugar chain present on a host cell, the inventors previously screened for hemagglutinin (HA)-binding peptides by the above-mentioned phage display library method, and reported that the peptide is capable of suppressing influenza infection by preventing intracellular invasion of an influenza virus (Japanese Laid-Open Patent Application No. 2002-284798, WO0/59932), revealing that peptides can be used to prevent influenza virus infection.

DISCLOSURE OF THE INVENTION

The present invention relates to influenza virus infection suppressors which contain as an active ingredient a sialylgalactose-binding peptide which binds to a sialylgalactose group (a group having the sialic acid-galactose structure), for example, a ganglioside GM3-binding peptide.

As described above, an influenza virus infects a host cell when the hemagglutinin in the viral membrane recognizes and binds to a sialic acid-containing sugar chain on the cell. The present invention is directed to suppressing influenza virus infection by blocking hemagglutinin receptors present on host cells. Specifically, the invention is directed to suppressing influenza virus infection by blocking the binding of an influenza virus to a host cell by using a peptide which binds to a sialylgalactose group present on the cell surface.

The present inventors previously found that hemagglutinin-binding peptides which bind to the influenza virus hemagglutinin are capable of suppressing influenza virus infection. These peptides inhibit the binding of influenza viruses to host cells by binding to the hemagglutinin present in the membrane of the influenza viruses.

Although the hemagglutinin-binding peptides are capable of acting on influenza viruses and thus preventing their infection, their effect is based on the premise that the influenza virus is present. This suggests that, for use in the clinical situation, the aforementioned peptides are effective when administered after influenza virus infection has been confirmed, as is the case with the sialidase inhibitor. The peptides are therefore expected to be effective as a therapeutic agent rather than a preventive agent. Based on a belief that compounds which act on a living body can be administered regardless of the presence or absence of influenza infection and thus produce a remarkable preventive effect, the inventors have assiduously studied on methods for suppressing influenza virus infection, and inferred that influenza virus infection can be suppressed by blocking a receptor on the host cell to which a hemagglutinin present in the influenza virus membrane binds. In this method, even if the influenza virus has not infected the subject, by blocking the receptor on the host cell in advance, the preventive effect on influenza virus infection can reasonably be expected. Further, in consideration of biocompatibility, half-life, ease of production, etc., it was also inferred that a compound having a rather low molecular weight would be suitable. However, in spite of the finding that sialylgalactose functions as a receptor during the process of influenza virus infection into animal cells, no compounds capable of acting on sialylgalactose and thus inhibiting influenza virus infection has previously been reported; the traditional idea has been that it is difficult to block the receptor with a single compound. Focusing on the structure of sialylgalactose, a receptor on the surface of host cells for influenza viruses, the inventors examined the effectiveness of peptides with ten and a few amino acids that bind to ganglioside GM3 (NeuAc$\alpha$2$\rightarrow$3Gal$\beta\rightarrow$4Glc$\beta$1$\rightarrow$1'Cer) which is involved in the structure of sialylgalactose. The inventors obtained peptides which can bind to ganglioside GM3, by targeting mouse B16 melanoma cells highly expressing the ganglioside GM3 on the cell surface with the phage display method. The inventors assiduously examined the obtained ganglioside GM3-binding peptides and found that, unexpectedly, the peptides solely prevent influenza from binding to the receptors on the cell surface and invading into cells.

Accordingly, the present invention encompasses the following:

(1) An influenza virus infection suppressor containing a sialylgalactose-binding peptide as an active ingredient;
(2) The influenza virus infection suppressor of (1), in which the sialylgalactose-binding peptide is a ganglioside GM3-binding peptide;
(3) The influenza virus infection suppressor of (1), containing the sialylgalactose-binding peptide of the following (a) or (b) as an active ingredient:

    (a) a peptide having the amino acid sequence of SEQ ID NO. 1 or 2;
    (b) a peptide having an amino acid sequence in which one or several nucleotides are deleted, substituted, or added, from/to the amino acid sequence of SEQ ID NO. 1 or 2, and having ganglioside GM3-binding activity;

(4) The influenza virus infection suppressor of any one of (1) to (3), in which the sialylgalactose-binding peptide is alkylated or lipidized;
(5) The influenza virus infection suppressor of any one of (1) to (3), in which the sialylgalactose-binding peptide is contained in a liposome;
(6) The influenza virus infection suppressor of any one of (1) to (5), in which the influenza virus is selected from the group consisting of type A influenza, type B influenza, and type C influenza;
(7) The influenza virus infection suppressor of any one of (1) to (5), in which the influenza virus is avian influenza or swine influenza;
(8) The influenza virus infection suppressor of any one of (1) to (7), further containing a pharmaceutically acceptable carrier;
(9) A method for preventing or treating influenza, including a step of administering the influenza virus suppressor of any one of (1) to (8) to a nonhuman animal;
(10) The method for preventing or treating influenza of (9), in which the nonhuman animal is a bird or a swine;
(11) A method for preventing or treating influenza, including the steps of preparing the influenza virus suppressor of anyone of (1) to (8) and administering the suppressor to a subject;
(12) The method for preventing or treating influenza of (11), in which the subject is selected from the group consisting of human, bird, and swine;
(13) Use of the influenza virus suppressor of any one of (1) to (8), for preventing or treating influenza; and
(14) Use of the ganglioside GM3-binding peptide of the following (a) or (b), for the manufacture of an influenza virus suppressor:

(a) a peptide having the amino acid sequence of SEQ ID NO. 1 or 2;

(b) a peptide having an amino acid sequence in which one or several nucleotides are deleted, substituted, or added, from/to the amino acid sequence of SEQ ID NO. 1 or 2, and having ganglioside GM3-binding activity.

BRIEF DESCRIPTION OF THE DRAWINGS

FIG. 1 shows the time course of phages that bind to a ganglioside GM3 monolayer fixed on a quartz-crystal microbalance.

FIG. 2 shows a result of an examination on binding selectivity of phage clones to the NeuAc-Gal structure.

FIG. 3 shows a result of an examination on the binding of phage clones to mouse melanoma B 16 cells by a flow cytometer.

FIG. 4 shows a result of an examination on recognition of a sialyloligosaccharide by c01-phages.

FIG. 5 shows the amount of binding of synthetic peptides to GM3 as a function of peptide concentration.

FIG. 6 shows an inhibition of the influenza virus infection to MDCK cells by peptide-containing liposomes.

FIG. 7 shows an inhibition of the influenza virus infection to MDCK cells by liposomes containing ganglioside GM3-binding peptides and liposomes containing hemagglutinin-binding peptides.

DESCRIPTION OF THE PREFERRED EMBODIMENT

The present invention is directed to infection suppressors which can be used to prevent and treat influenza virus infection and which contain a sialylgalactose-binding peptide, for example, a ganglioside GM3-binding peptide, as an active ingredient.

A sialylgalactose group, having a structure in which a sialic acid and a galactose are bound, serves as a receptor for hemagglutinin when an influenza virus penetrates cells. Accordingly, sialylgalactose-binding peptides are capable of suppressing influenza infection into cells by preventing an influenza virus from binding to the cells via a sialylgalactose group. Examples of compounds having a sialylgalactose group include ganglioside GM3. The sialylgalactose-binding peptide according to the present invention includes the ganglioside GM3-binding peptide.

It is noted that the peptide according to the present invention is not particularly limited as long as it can bind to a sialylgalactose group; the peptide can suppress an influenza virus infection to a cell as long as the cell expresses a sialylgalactose group on its surface even if a ganglioside GM3 is not expressed on the surface.

Influenza viruses to be suppressed their infection by the infection suppressor according to the present invention are spherical RNA viruses with diameter of about 100 nm, belonging to the Orthomyxoviridae family, and their types are not limited. The influenza virus suppressor according to the present invention can suppress an influenza infection regardless of the types of the influenza because it blocks a receptor on the cell recognized by the influenza. The types of influenza viruses include type A (H2N2, H3N2, H1N1, etc.), type B, type C, human isolates, avian isolates (avian influenza viruses (H5N1, H7N2, H7N7, etc.)), swine isolates (swine influenza viruses), and other mammal isolates (e.g., equine isolates). The influenza virus suppressor is capable of extensively suppressing all these types of influenza. Some influenza viruses have a number of subtypes depending on the serotypes of hemagglutinin and sialidase (neuraminidase). The influenza virus infection suppressor according to the present invention can suppress influenza virus infection regardless of the type or subtype.

Further, the influenza virus infection suppressor according to the present invention can be used for any animal as long as it is an animal which influenza viruses can infect, and thus can suppress infections of humans, birds, swine, etc. by the influenza viruses.

The sialylgalactose-binding peptide according to the present invention can be selected by the phage display library method using the known phage display library as described below. The phage display library method can be performed by, for example, the method of Scott and Smith (Scott, J. M. and Smith, G. P., Science, 249, 386-390 (1990); Smith, G. P. and Scott, J. K., Methods in Enzymology, 217, 228-257 (1993)). Alternatively, it can be performed according to the description in Japanese Laid-Open Patent Application Nos. 2000-253900 and 2002-284798.

The phage display library to be used can be a commercially available one.

First, random DNA sequences are inserted into a known phage display library and they are constructed such that peptides having a random amino acid sequence can be expressed on the surface of the outer envelope surface of the phages. In this case, display phages to be used may be the ones, for example, which have been constructed such that random pentadecapeptides can be expressed on the surface of the outer envelope surface of the phages by inserting random DNA into the coat protein pIII gene of the phage, as shown in the Examples. By using display phages, those phages each expressing about $10^8$ types of peptides can be obtained.

Next, phages randomly expressing peptides are panned with ganglioside GM3. For this panning, a ganglioside GM3 monolayer may be prepared on a suitable substrate (ganglioside GM3 monolayer built-up substrate), and the phages that bind to the monolayer can be selected. The phages selected are infected into E. coli, mass cultured, isolated, and purified, to obtain peptide-expressing phages which bind to ganglioside GM3. By repeating the panning operation several times, phages capable of expressing peptides capable of binding specifically to ganglioside GM3 can be selected and concentrated.

Then, by extracting and sequencing DNA from the phages selected, peptides which specifically bind to the sialylgalactose group can be obtained. DNA sequencing may be performed by the known methods such as Maxam-Gilbert method, and a commercially available sequencer may be used.

When using a monolayer in this manner, since only sugar chains protrude on the surface, sialylgalactose-binding peptides can be efficiently selected. It is noted that when a monolayer is prepared and peptide-expressing phages which bind to ganglioside GM3 are selected, a quartz crystal oscillator, known as the microbalance, can be used. By using a quartz crystal oscillator, the weight changes at the surface of the gold electrode on the quartz crystal oscillator caused by the binding of phages can be detected as frequency changes. The method based on the quartz crystal oscillator can be performed in accordance with the method described in, e.g., Biochim. Biophys. Acta., 1138, 82-92 (1998), or Biochim. Biophys. Acta, 1285, 14-20 (1996). Specifically, the surface of the electrode on the oscillator is attached horizontally to the surface of the monolayer prepared on Tris-buffered saline (TBS), which serves as the water subphase, in a trough, and oscillated. When the oscillation becomes stable, a phage library solution is added and the oscillation frequency corresponding to the binding of phages is measured. The presence or absence of binding can be thus detected.

Examples of sialylgalactose-binding peptides include the ganglioside GM3-binding peptides having 15-mer amino acid sequence shown in SEQ ID NOs. 1 and 2. Each of these peptides can be used as an active ingredient of the influenza virus infection suppressor according to the present invention. Alternatively, peptides containing an amino acid sequence shown in SEQ ID NO. 1 or 2, from/to which one or several nucleotides are deleted, substituted, or added, and capable of binding to ganglioside GM3 can be used as an active ingredient in the influenza virus infection suppressor according to the present invention. The above-mentioned "one or several" refers preferably to 1 to 10, more preferably to 1 to 5, 1 to 4, 1 to 3, 1 to 2, or 1. Alternatively, a peptide which consists of an amino acid sequence encoded by a DNA that can hybridize, under stringent conditions, to a DNA complementary to the DNA encoding the peptide having the amino acid sequence shown in SEQ ID NO. 1 or 2, and which can bind to ganglioside GM3, maybe used as an active ingredient in the influenza virus suppressor according to the present invention. "Stringent conditions" as described herein include a condition under which a DNA can be identified by hybridization in the presence of 0.7 to 1.0 M NaCl at 68°C by using the filter with DNA immobilized thereon and then washing the filter at 68°C using 0.1 to 2-fold concentrated SSC solution (a 1-fold concentrated SSC solution consising of 150 mM NaCl and 15 mM sodium citrate). Alternatively, a peptide which has an amino acid sequence encoded by a degenerate mutant of the DNA encoding the peptide having the amino acid sequence shown in SEQ ID NO. 1 or 2, and which can bind to ganglioside GM3, may also be used as an active ingredient in the influenza virus suppressor according to the present invention. Whether or not a peptide binds to ganglioside GM3 can be examined, for example, by the above-mentioned quartz crystal oscillator to which a ganglioside monolayer has been attached.

The above-mentioned peptides can be synthesized by known liquid-phase and the solid-phase peptide synthesis methods. Alternatively, the peptides can also be prepared with selected phages and E coli. The peptides obtained in this manner may be purified by known peptide purification methods.

A modified peptide of the ganglioside GM3-binding peptide may also be used. Since the modification of a peptide can enhance its hydrophilicity, prolong its blood half-life, and also enhance cell affinity and/or tissue affinity, the modified peptide can be expected to produce a greater effect as an influenza infection suppressor. Further, since a polymerization of the peptide by the modification sterically impairs the binding of an influenza virus to ganglioside GM3, a greater effect can be expected as a suppressor.

Examples of peptide modifications include alkylation, lipidation, and binding of water-soluble polymers such as PEG.

Binding of an alkyl group can be performed by the known methods. For example, alkylamine may be bound to the C-terminal carboxyl group of a ganglioside GM-binding peptide, or a fatty acid may be bound to the N-terminal amino group. The binding of alkylamine to the terminal carboxyl group or the binding of a fatty acid to the terminal amino group can be performed by the amide bond forming reaction. Examples of the alkyl group to be bound include, but are not limited to, an alkyl group having 2 to 20 (e.g., 18) carbon atoms. The fatty acid to be used for binding is also not limited, but a fatty acid present in the living body can suitably be adopted. Specific examples include fatty acids having about 12 to 20 carbon atoms: saturated fatty acids such as lauric acid, myristic acid, palmitic acid, stearic acid, and arachic acid, etc.; and unsaturated fatty acids such as oleic acid, elaidic acid, linolic acid, linolenic acid, and arachidonic acid, etc.

Lipidation of a ganglioside GM3-binding peptide can also be performed by the known methods, for example, in accordance with the descriptions in "New Current, 11 (3), 15-20 (2000); Biochemica et Biophysica Acta., 1128, 44-49 (1992); FEBS Letters, 413, 177-180 (1997); and J. Biol. Chem., 257, 286-288 (1982), etc. Specifically, a ganglioside GM3-binding peptide can be bound with various phospholipids via the 2-hydroxyl group or 3-phosphate group of the phospholipids. A suitable spacer may be used for this binding. Various kinds of condensation methods can be adopted for the reactions. One example is the method using a reactive SH group, in which an amino acid sequence of suitable length, having a few amino acids including a cysteine, is bound to the N-terminus or the C-terminus of a ganglioside GM3-binding peptide. Examples of the phospholipid to be used include, but are not limited to, phosphatidic acid, phosphatidylcholine (lecithin), phosphatidylethanolamine, phosphatidylserine, phosphatidylinositol, and phosphatidylglycerol, etc. Lipidized peptide thus obtained are referred to as a lipopeptide.

Examples of the water-soluble polymer to be used when the peptide is modified by binding of the water-soluble polymer include, polyethylene glycol, monomethoxy-polyethylene glycol, dextran, pnly(N-vinyl pyrrolidone)polyethylene glycol, propylene glycol homopolymers, polypropylene oxide/ethylene oxide copolymers, and polyvinyl alcohol, etc. These polymers can bind covalently to the $\alpha$-amino group of the N terminus or the $\varepsilon$-amino group of a lysine of the protein via a reactive group such as aldehyde. Preferred among these polymers is PEG, and the preferred molecular weight of PEG is 6 kDa to 50 kDa.

Another example of the method for polymerizing a ganglioside GM3-binding peptide is dendrimerization.

Additionally, a liposome preparation containing a ganglioside GM3-binding peptide can also be used as the influenza infection suppressor according to the present invention. The term "liposome" refers to a membranous closed vesicle composed of an aggregated lipid layer in a membrane form and an inner water phase. The liposome preparation according to the present invention can be prepared by making a liposome contain a ganglioside GM3-binding peptide. The liposomes include liposomes in which lipid phospholipids constitutes the membrane, and liposomes in which neutral and acid phospholipids constitute the membrane.

Examples of the acid phospholipids as a constituent of the membrane include natural or synthetic phosphatidylglycerols (PGs) such as dilauroylphosphatidylglycerol (DLPG) dimyristoylphosphatidylglycerol (DMPG), dipalmitoylphosphatidylglycerol (DPPG), distearoylphosphatidylglycerol (DSPG) dioleoylphosphatidylglycerol (DOPG), egg yolk phosphatidylglycerol (egg yolk PG), hydrogenated egg yolk phosphatidylglycerol; and natural or synthetic phosphatidylinositols (PIs) such as phosphatidylinositol (DLPI), dimyristoylphosphatidyl inositol (DMPI), dipalmitoylphosphatidylinositol (DPPI), distearoylphosphatidylinositol (DSPI), dioleoylphosphatidylinositol (DOPI), soybean phosphatidylinositol (soybean PI), and hydrogenated soybean phosphatidylinositol. Each of these constituents may be used alone or in combination of two or more.

Examples of the neutral phospholipids include natural or synthetic phosphatidylcholines (PCs) such as soybean phosphatidylcholine, egg yolk phosphatidylcholine, hydrogenated soybean phosphatidylcholine, hydrogenated egg yolk phosphatidylcholine, dimyristoylphosphatidylcholine (DMPC), dipalmitoylphosphatidylcholine (DPPC), dilauroylphosphatidylcholine (DLPC), distearoylphosphatidylcholine (DSPC), myristoylpalmitoylphosphatidylcholine (MPPC), palmitoylstearoylphosphatidylcholine (PSPC) and dioleoylphosphatidylcholine (DOPC); and natural or synthetic phosphatidylethanolamines (PEs), such as soybean phosphatidylethanolamine, egg yolk phosphatidylethanolamine, hydrogenated soybean phosphatidylethanolamine, hydrogenated egg yolk phosphatidylethanolamine, dimyristoylphosphatidylethanolamine (DMPE), dipalmitoylphosphatidylethanolamine (DPPE), dilauroylphosphatidylethanolamine (DLPE), distearoylphosphatidylethanolamine (DSPE), myristoylpalmitoylphosphatidylethanolamine (MPPE), palmitoylstearoylphosphatidylethanolamine (PSPE) and dioleoylphosphatidylethanolamine (DOPE). Each of these constituents maybe used alone or in combination of two or more.

Liposomes containing a ganglioside GM3-binding peptide may be prepared in accordance with the known methods using the aforementioned phospholipids. In this preparation, acid phospholipid is contained, by proportion, about 0.1 to about 100 mol%, preferably about 1 to about 90 mol%, more preferably about 10 to about 50 mol%, in the liposome membrane constitutes.

Cholesterol etc. may be added when preparing the above-mentioned liposomes. By adding cholesterol, the fluidity of phospholipid can be adjusted so that liposomes can be prepared more simply and conveniently. Typically, cholesterol is added and mixed at or up to an equal volume to phospholipid, preferably in a 0.5 to 1-fold volume.

The mixing ratio of an acid phospholipid to the ganglioside GM3-binding peptide in a liposome dispersion is typically about 0.5 to about 100 equivalents of acid phospholipid per equivalent of peptide, preferably about 1 to about 60 equivalents of acid phospholipid per equivalent of peptide, and more preferably about 1.5 to about 20 equivalents of acid phospholipid per equivalent of peptide.

The ratio of the ganglioside GM3-binding peptide content in a liposome is typically a few to a few tens of mol%, preferably 5 to 30 mol%, and more preferably 5 to 10 mol% in all the lipids.

A liposome preparation containing the ganglioside GM3-binding peptide according to the present invention may be manufactured by the known methods.

For example, multilayer vesicles (MLVs) are prepared as follows. First, the lipid is dissolved in an organic solvent (chloroform, ether, etc.), and then put in a round bottom flask. The organic solvent is removed under a nitrogen current or under reduced pressure to form a thin lipid film at the bottom of the flask. Optionally, the flask may be left in a desiccator under reduced pressure for complete removal of residual solvent. Subsequently, the lipid is hydrated by adding a drug solution onto the thin lipid film to yield a turbid, opalescent liposome suspension.

Large unilamellar vesicles (LUVs) can be produced by adding $Ca^{2+}$ to small unilamellar phosphatidylserine vesicles, which are fused to form a cylindrical sheet, followed by addition of the chelating agent EDTA to remove the $Ca^{2+}$ (Biochim. Biophys. Acta 394, 483-491, 1975). LUVs can also be produced by injecting the lipid in ether into an aqueous solution at about 60°C to evaporate the ether (Biochim. Biophys. Acta 443 629-634, 1976).

Alternatively, the liposome preparation method using the reverse-phase technique devised by Szoka et al. (Proc. Natl. Acad. Sci. U. S. A, 75, 4194-4198, 1978) may be adopted. According to this method, by adding a drug solution to an ether solution of phospholipid and sonicating the resulting mixture, a W/O type emulsion is formed. Ether is removed from this W/O type emulsion in an evaporator under reduced pressure. Then a buffer is added and the resulting emulsion is stirred on a vortex mixer, resulting in a phase inversion from the W/O type emulsion to an O/W type emulsion. By removing the residual solvent, a liposome can be obtained.

In addition to these methods, a liposome with small particle-size can be prepared by the French press method (FEBS lett. 99, 210-214, 1979). Alternatively, the freeze-drying method for a liposome with a high capacity (Chem. Pharm. Bull, 32, 2442-2445, 1984), and the freezing-thawing method (Chem. Pharm. Bull. 33, 2916-2923, 1985), both of which were reported by Ohsawa et al., may also be adopted.

The liposomes thus prepared can be made uniform in terms of particle size by dialysis (J. Pharm. Sci. 71, 806-812, 1982) or the filtrating method using a polycarbonate membrane (Biochim. Biophys. Acta 557, 9-23, 1979; Biochim. Biophys. Acta 601, 559-571, 1980). To remove the drug which has not been retained in the liposome from the prepared liposome solution, the dialysis, a gel filtration method, and a centrifugation method can be adopted (see Liposome. "Shishitsu no Kagaku" (Chemistry of lipids); Biochemical Experiment Lecture 3, edited by the Japanese Biochemical Society, published by Tokyo Kagaku Dojin, 1974). Further, the liposome can be also concentrated by using the dialysis membrane.

The liposome dispersion thus prepared may appropriately contain, as a required additive for formulating the preparation, various kinds of known substances, such as antiseptics, isotonizing agents, buffers, stabilizers, solubilizing agents, and absorption accelerators, etc. The liposome dispersion can also be diluted with water or a solution containing these additives, as required. Specific examples of the above-mentioned additives include: preservatives, which are effective for fungi and bacteria, such as benzalkonium chloride, benzethonium chloride, chlorhexidine, parabens (methyl paraben, ethyl paraben, etc.), and thimerosals; isotonizing agents, such as D-mannitol, D-sorbitol, D-xylitol, glycerol, glucose, maltose, sucrose, and electrolytes (e.g., polyhydric alcohols and sodium chloride); and stabilizers, such as tocopherol, burylhydroxyanisole, butylhydroxytoluene, ethylenediaminetetraacetic acid salt (EDTA), and cysteine.

Additionally, another agent, for example, an antiviral agent, may be further encapsulated into the above-mentioned liposome containing the HA-binding peptide according to the present invention, and a liposome preparation may be similarly manufactured.

The liposome preparations may be manufactured in accordance with the methods specifically described by, for example, Woodle et al. (Long Circulating Liposomes: old drug, New therapeutic., M. C. Woodle, G. Storm, Eds: Springer-Verlag, Berlin (1998)) or Namba et al. (Liposomal applications to cancer therapy, Y. Namba, N. Oku, J. Bioact. Compat. Polymers, 8, 158-177 (1993)).

For the manufacture of the liposome preparations, the above-mentioned alkylation peptide and/or lipidized peptide may be used as a lipid component.

The amount of the ganglioside GM3-binding peptide in a pharmacological compositions including the above-mentioned liposome preparations according to the present invention is not limited but may be appropriately selected depending on the subject to whom the composition is administered: typically the order of 0.0002 to 0.2 (w/v%), preferably the order of 0.001 to 0.1 (w/v%) in the composition.

The influenza virus infection suppressor according to the present invention can prevent influenza virus infection by being administered to a subject who has not been infected with an influenza virus, and can also be used as a therapeutic agent against the influenza viruses because it can suppress an influenza virus infection into other cells in the body by being administered to an already infected subject. The influenza infection suppressor according to the present invention may contain a pharmaceutically acceptable carrier, a diluent, and an excipient, together with the ganglioside GM3-binding peptide as an active ingredient. For aqueous preparations, purified water (sterilized water), a physiological buffer solution, an isotonic solution, etc. may be used as a carrier. Glycol, glycerol, an injectable organic ester such as olive oil, etc. may also be used. For tablets, gelatinizer, lactose, magnesium stearate, etc. may be used as a carrier or an excipient.

The influenza virus infection suppressor according to the present invention can be administered in various dosage forms as a pharmaceutical composition. Such dosage forms include oral preparations such as tablets, capsules, granules, powders, syrups, etc.; or parenteral preparations such as injections, infusions, suppositories, etc. Sprays are another alternative. For sprays, a solution containing the influenza virus suppressor according to the present invention may be sprayed orally or pernasally to the subject. Such sprays can be efficiently administered to domestic animals, such as birds and swine as well. These compositions are manufactured by the known methods and generally used in the pharmaceutical field.

The administration routes for the influenza virus infection suppressor according to the present invention include, but are not limited to, oral administration, intravenous injection, intramuscular injection, intradermal injection, subcutaneous injection, intraperitoneal injection, nebulization, etc. Alternatively, the influenza virus suppressor according to the present invention can also be administered by mixing into the drinking water, feed, etc. for domestic animals such as birds, swine, etc. The doses may be appropriately determined depending on the severity of the patient, and a pharmaceutically effective dose of the composition according to the present invention is administered to the patient. "To administer a pharmaceutically effective dose" as described herein refers to administering a drug at a level appropriate for the treatment of each disease. The number of administration of the composition according to the present invention may be appropriately selected depending on the symptom of the patient. For example, the dose can be selected in the range equivalent to about 0.001 to 100 mg/day for adult patients of the amount of a ganglioside GM3-binding peptide. The preparation according to the present invention can be administered as, but not limited to, a once daily dose, and can also be administered as multiple doses. When administered to a domestic animal such as a bird or a swine, the dose can be appropriately determined in consideration of the body weight etc.

The influenza virus infection suppressor according to the present invention may contain another compound capable of suppressing infection by the influenza virus as an active ingredient. Examples of such compound include the influenza virus hemagglutinin-binding peptide described in Japanese Laid-Open Patent Application No. 2002-284798. Specifically exemplified is the peptide as described in Japanese Laid-Open Patent Application No. 2002-284798 and WO00/59932. By making an influenza virus suppressor contain a ganglioside GM3-binding peptide and an influenza virus hemagglutinin-binding peptide, both the site present on the influenza virus for binding to cells and the site present on cells for binding to the influenza virus can be blocked, and a greater effect for suppressing influenza virus can be expected. The influenza virus hemagglutinin-binding peptide, like the above-mentioned ganglioside GM3-binding peptide, can also contain the partially mutated amino acids, as well as the modified amino acids. The influenza virus hemagglutinin-binding peptide may be contained in a liposome. When the influenza virus infection suppressor according to the present invention is a liposome preparation, it may contain both a ganglioside GM3-binding peptide and an influenza virus hemagglutinin-binding peptide.

Next, the present invention will be explained in more detail by referring to Examples.

EXAMPLES

Procurement of materials

Ganglioside GM3 (NeuAcα2 → 3Galβ1 → 4Glcβ1 → 1'Cer) was obtained from Snow Brand Milk Products Co., Ltd. (Japan). 6' GM3 (NeuAcα2 → 6Ga1β1 → 4Glcβ1 → 1'Cer), a synthetic GM3 analog, was prepared by the method as described in reference (42). Lactosyl ceramide (LacCer), galactosyl ceramide (GalCer), and glucocerebroside (GlcCer) were purchased from Sigma Chemical Co. (St Louis, MO, USA). Egg yolk phosphatidylcholine (egg PC) and cholesterol were obtained from NOF Corporation Inc. (Japan) and Nacalai Tesque Inc. (Japan), respectively.

A phage display 15-mer random peptide library (with a diversity of $10^8$) was obtained in accordance with the description in reference (43). The 15-mer peptide amide (peptide-$NH_2$) and N-stearoyl (octadecanoyl) peptide amide ($C_{17}H_{35}CO$-peptide-$NH_2$) were synthesized by the standard Fmoc method with the automatic peptide synthesizer ACT357 from Advanced Chemtech. The peptide amide and N-stearoyl peptide amide were purified by reversed-phase high-performance liquid chromatography. Next, their purity and the expected structures were confirmed by MALDI-TOF/MS.

Mouse B16 melanoma cells were obtained from the RIKEN Cell Bank (Japan). Madin-Darby canine kidney (MDCK) cells and influenza virus A/Puerto Rico/8/34 strain (H1N1) were offered by K. Nagata (University of Tsukuba, Japan).

EXAMPLE 1

Selection of the ganglioside GM3-binding peptides

(1) Affinity selection of sialylgalactose-binding peptides

A lipid solution (chloroform/methanol = 2:1, v/v) containing ganglioside GM3 (ca. 0.5 mg/mL) was spread on Tris-buffered saline (TBS) (50 mM Tris-HCl, 150 mM NaCl, pH 7.5) in a Teflon-coated Langmuir trough (USI Co., Ltd. (Japan)). The temperature of the water subphase was held at 20°C. The surface pressare-area (π-A) isotherms were monitored by the Wilhelmy plate method. The GM3 monolayer was compressed at a constant rate (10 cm$^2$ min$^{-1}$) and horizontally transferred to the gold surface of a quartz-crystal microbalance (QCM; 9 MHz, AT-cut, 4.5 mm in diameter,

15.9 mm$^2$ in area) at a surface pressure of 30 mN m$^{-1}$ (Reference (21)). The QCM was transferred to a handmade plastic tube filled with 1 ml of TBS, and the temperature of the buffer was maintained at 20°C while stirring. Phages (6.1 to 87 x 10$^{10}$ transducing units at each selection round) were injected into a cuvette. To determine the incubation time, the binding of the phages to the monolayer ($\Delta$F, Hz) was monitored (reference (35)). The binding of the phage became almost equilibrated in 15 min (data not shown). The QCM was picked up from the buffer and washed three times with TBS. The phages bound to the monolayer were eluted with 0.1 N Gly-HCl buffer (pH 2.2) for 15 min. The eluate was neutralized with 1 M Tris-HCl buffer (pH 9.1), and then, the obtained phages were amplified by infecting host bacterial cells of E. coli K91Kan. This process was repeated four times to amplify GM3-specific phages. Individual phage clones were isolated, amplified and precipitated with polyethylene glycol/NaCl (PEG #6000) (reference (26)). DNA of each phage clone was purified with a QIAprep Spin M13 kit (QIAGEN) and used as a template for sequencing to infer amino acid alignments.

(2) Assay of phage clone binding ability by ELISA

A polystyrene multiwell plate had been blocked with 1% BSA/TBS and washed 3 times in advance. A plastic plate (13.5 mm in diameter; Sumitomo Bakelite Co., Ltd., Japan) was horizontally attached to the ganglioside GM3 or 6' GM3 monolayer prepared as described above. The phage clones (0.01 to 10 nM in 200 $\mu$l of TBS) were incubated with the monolayer at 4°C for 30 min. The other side of the plastic plate was blocked by adding 0.5% BSA/TBS, and, subsequently, washed twice with 0.5% BSA/TBS. Bound phages were incubated with 1:2000 (v/v) diluted anti-fd bacteriophage (Sigma) at 4°C for 1 hour, and, subsequently, labeled with 1:2000 (v/v) diluted anti-rabbit immunoglobulin G peroxidase conjugate at 4°C for an hour. Color was developed with o-phenylenediamine and detected at 492 nm. Each experiment was performed three times. The increase in absorbance ($\Delta$A at 492nm) showed a simple saturation curve versus phage concentration, reflecting a linear relationship plated between [phage]/$\Delta$A and [phage] as in the following equation:

$$[phage]/\Delta A = [phage]/\Delta amax + K_d/\Delta Amax \qquad (I)$$

A maximum absorbance ($\Delta$Amax) and a dissociation constant ($K_d$) were calculated from the gradient and the intercept of the equation, respectively. $K_d$ was in the range of 0.018 to 0.091 nM. $\Delta$Amax of control phages was also assayed each time and relative binding affinity ($\Delta$Amax/$\Delta$Amax, control) was evaluated (Table 1). Wild-type phages were used as the control phages. (3) Cells

Mouse B16 melanoma cells were grown in Dulbecco's Modified Eagle's Medium (ICN Biomedicals), supplemented with 10% fetal bovine serum (Life Technologies, Inc.), 100 units/ml penicillin G, and 100 units/ml streptomycin, under the condition of 37°C, 5%CO$_2$, 95% air. Madin-Darby canine kidney (MDCK) cells were grown in Minimum Essential medium eagle (MEM; GIBCO BRL), supplemented with 10% fetal bovine serum, 0.1% NaHCO$_3$, 10 $\mu$g/ml glutamine, 100 units/ml penicillin G, and 100 units/ml streptomycin under the condition of 37°C, 5%CO$_2$, 95% air.

(4) Flow cytometry

Trypsin-treated B16 cells (2 x 10$^5$) were incubated on ice for 0.5 hour with 200 $\mu$l of phage clones ([phage] = 0.1 to 500 nM) serially diluted in 1% BSA/phosphate buffered saline (PBS). After three washes with 1% BSA/PBS, the cells were incubated on ice for 0.5 hour with 200 $\mu$l of 400-fold-diluted (v/v) rabbit anti-fd bacteriophage antibody (Sigma). Following two washes, the cells were incubated on ice for 0.5 hour with 200 $\mu$l of 400-fold-diluted (v/v) anti-rabbit IgG fluorescein isothiocyanate (FITC) conjugate (Sigma). The FITC-labeled cells were washed twice with 1% BSA/PBS and analyzed in a flow cytometer (EPICS XL; Coulter).

(5) Inhibition by addition of a monosaccharide

Trypsin-treated B16 cells (2 x 10$^5$) were incubated on ice for 1 hour with 200 $\mu$l of phage clones ([phage] =10 nM) in the presence or absence of a monosaccharide (1 or 5 mM N-acetyl neuraminic acid (NeuAc), glucose (Glc), or glucuronic acid (GlcU)). The cells were washed, labeled with FITC, and analyzed by a flow cytometer.

(6) Removal of sialic acid from glycoproteins on cells

Trypsin-treated B16 cells (2 x 10$^5$) were incubated at 37°C for 1.5 hours (pH 6.5 or 7.4) with 0.05 unit of neuraminidase derived from Arthrobacter ureafaciens (Sigma). After three washes with 1% BSA/PBS, the cells ware incubated with phage clones ([phage] - 10 nM), labeled with FITC, and analyzed in the flow cytometer.

(7) Analysis of the interaction between synthetic peptides and a glycolipid monolayer by using a quartz-crystal microbalance (QCM)

The monolayer of glycolipids (GM3, 6'GM3, LacCer, GalCer, or GlcCer) was prepared on a Langmuir-type trough and horizontally transferred to QCM (27 MHz, AT-cut, 2.5 mm in diameter, and 4.9 mm$^2$ in area). The 27-MHz QCM showed a higher sensitivity than the 9-MHz QCM (about 10-fold) (references (44) - (46)). Solutions of 0.1 mM, 1 mM, and 10 mM peptide in TBS were added to the cell cuvette of the QCM apparatus, and decreases in frequency ($\Delta F$, Hz) in response to the addition of the peptides were monitored. Each experiment was performed 2 to 6 times. The relationship between the binding amount ($\Delta m$, ng cm$^{-2}$) and the $\Delta F$ was obtained by a Sauerbrey equation (reference (47)). In these experiments, 1 Hz of $\Delta F$ corresponded with a mass increase by 1.1 ng cm$^{-2}$ of peptide. $\Delta m$ was plotted versus peptide concentration (final concentration: 1 to 70 $\mu$M). Next, the maximum binding amount ($\Delta m_{max}$) and the dissociation constant ($K_d$) were calculated from the following equation described in references (44-45, 48).

$$[\mathrm{peptide}]/\Delta m = [\mathrm{peptide}]/\Delta m_{max} + K_d/\Delta m_{max} \qquad (II)$$

(8) Preparation of a liposome containing the peptide

Mixed lipid of egg yolk phosphatidylcholine (PC) and cholesterol (2:1, molar ratio) dissolved in chloroform/methanol (2:1, v/v) were added to a round bottom flask and evaporated to yield a thin lipid film. The lipid film was dried in a vacuum overnight. The film was swollen with PBS (pH 7.4) on the vortex and sonicated for 30 min. The PC / cholesterol liposome was mixed with an aqueous solution of N-stearoyl peptide amide. The final molar ratio of PC: cholesterol:peptide was 20:10:3. The following results were obtained in Example 1.

(a) Phage display selection for a GM3 monolayer

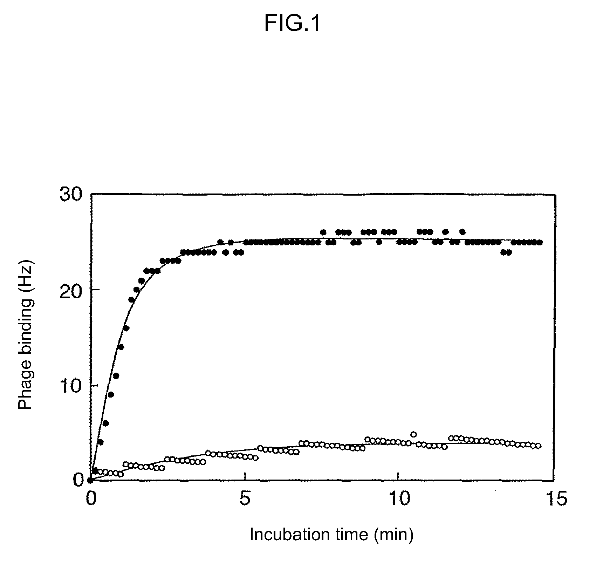

Peptide sequences that bind to the sugar chains of GM3 were selected by the method for phage library bound to a lipid monolayer as described in reference 35. A CM3 monolayer was prepared at an air-water interface and used for affinity selection. GM3-specific phages were increased through the affinity selection process repeated four times. The relative yield of the recovered phages was increased to 0.2 to 15 x 10$^6$ through the four rounds of biopanning (data not shown). The binding affinity of the selected phages for the GM3 monolayer was confirmed by using a 9-MHz quartz-crystal microbalance (QCM) (references (21)-(22)). QCM frequency decreases (mass increases) in response to the addition of phages (10$^{10}$ TU/mL) in Tris-buffered saline (50 mM Tris-HCl, 150 mM NaCl, pH 7.5) were monitored (FIG. 1).
Fig. 1 shows a result of the phage library (white circles) and the phage clones in the fourth round of the affinity selection by using the GM3 monolayer (black circle). The GM3 monolayer was attached to the gold surface of the 9-MHz QCM, and the QCM was immersed in 1 ml of Tris-buffered saline at pH 7.5. A phage solution containing 6 x 10$^{10}$ transducing units was injected into the buffer, and frequency decreases in response to the phage binding were plotted versus time. This result showed that the final phage concentration was 2 nM.
The frequency changes showed that the binding amount of selected phages (26 Hz) was 6.5-fold larger than that of the initial library (4 Hz). These results indicate that the affinity selection yielded the GM3-binding phages from random phage libraries.
Twenty-seven GM3-specific phage clones were isolated and their DNA sequences were determined. The deduced amino acid sequences representing randomized regions indicate that seven kinds of 15-mer sequences were found as shown in Table 1 (sequences 1 to 7). The sequences were divided into two groups, each of which had a deduced consensus motif, W-xxxA-R or WRx-VxFxS. Each consensus motif consisted of Arg (R), Trp (W), and Phe (F). Interestingly enough, the GM1-binding peptide consensus motif also contained these three amino acids (arginine and aromatic amino acids) (reference 35). Further, the c01 sequence selected in this study had the same sequence as one of GM1-binding peptides (GM1/peptide 2). This is due to the limitation of the library diversity, as the same phage library was used. These results suggest that the c01-sequence has an extensive affinities to sialic acid-containing glycolipids.

Table 1

Deduced peptide sequences of and binding affinities of phage clones isolated after four rounds of affinity selection.

| Clone No. | Peptide sequence[a] | Frequency[b] | Relative binding affinity[c] |
| --- | --- | --- | --- |
| c01 | G**WW**YKGR**AR**PVSAVA | 15/27 | 2.1 |

(continued)

| Clone No. | Peptide sequence[a] | Frequency[b] | Relative binding affinity[c] |
|---|---|---|---|
| | Deduced peptide sequences of and binding affinities of phage clones isolated after four rounds of affinity selection. | | |
| c07 | LS**W**PLH**A**G**R**GFRWVS | 1/27 | 1.2 |
| c21 | G**W**YSSRHYVRSLNGL | 1/27 | 1.1 |
| c11 | QQLVYN**WW**AVSS**A**R**R** | 1/27 | 1.1 |
| c13 | RAV**WR**HS**V**ATP**S**HSV | 7/27 | 1.8 |
| c15 | L**WR**P**VLF**H**S**AVRALG | 1/27 | 1.7 |
| c30 | **WR**GVY**F**GDRWLGSQP | 1/27 | 1.4 |

a. Deduced amino acid sequences; deduced consensus motifs are denoted by bold letters.

b. The number of the phage clones isolated.

c. Ratio of $\Delta$Amax / $\Delta$Amax, control. The binding of the phage clones to GM3 was evaluated by ELISA. The N-terminal sequence of the control phage clones (wild type) is AETVESCLAKPHTEN.

(b) Affinity screening by ELISA

Binding affinity of isolated phage clones for GM3 was evaluated by ELISA. A GM3 monolayer was prepared at an air-water interface and it was transferred to a plastics plate. The plate was transferred to a 24-well multiplate, then incubated with the phage clones for 30 min. A saturation curve was obtained from the plot of absorption of the enzymatic coloring vs. phage concentration. The maximum absorbance ($\Delta$Amax) was calculated from the aforementioned equation I. The results obtained form the ELISA showed that seven phage clones can strongly bind to GM3 in the following order: c01 > c03 > c15 > c30 > c07 > c21 = c11. Relative binding affinity, i.e., ($\Delta$Amax of the selected phages) / ($\Delta$Amax of the control phages displaying AETVESCLAKPHTEN) was between 1.1 and 2.1 (Table 1). The phage clone c01, which appeared most frequently (15/27), exhibited the highest affinity to GM3.

(c) Binding affinities of phage clones

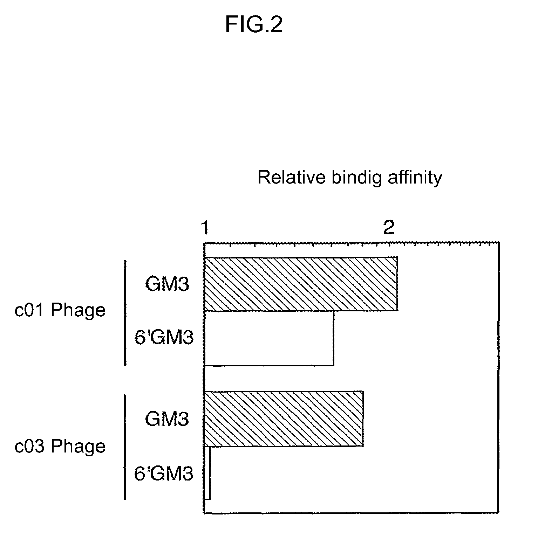

The binding affinities of the two phage clones, c01 and c03, were further examined by ELISA. The binding affinities of these clones were assayed by using GM3 containing NeuAc$\alpha$2→3Gal binding and synthetic 6'GM3 containing NeuAc$\alpha$2→6Gal bond. The c01 clone was bound to both GM3 and 6'GM3 with affinities 2.1-fold and 1. 6-fold, respectively, higher than the control clones, (FIG. 2).

The isolated c01 and c03-phage clones were serially diluted (0.05 to 10 nM) and the amounts of the bound phage clones, in interaction with GM3 (NeuAc$\alpha$2→3Gal$\beta$1→4G1c$\beta$1→1'Cer) or 6'GM3 (NeuAc$\alpha$2→6Gal$\beta$1→4Glc$\beta$1→1'Cer) monolayer, were evaluated by the ELISA method. Relative binding affinities ($\Delta$Amax/$\Delta$Amax$_{control}$) were obtained by dividing the maximum absorbance of the phage clones ($\Delta$Amax) by the maximum absorbance of the control phages ($\Delta$Amax$_{control}$).

The c01-phage clone was shown to bind to both of the NeuAc-Gal bonds, whereas the c03-clone bound specifically to GM3 by 1.8-fold higher than the control clone. The c03-phage clone exhibited different binding affinities between the $\alpha$2→3 and $\alpha$2→6 bonds of sialic acid to galactose.

(d) Specific binding to the sialyloligosaccharides in glycoprotein on B16 cells

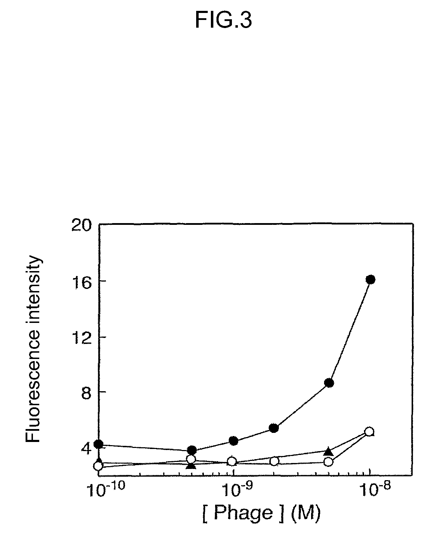

The binding affinities of the phage clones selected for animal cells were examined. Mouse B16 melanoma cells have two kinds of sialosyl-galactose bonds ($\alpha$2→3 and $\alpha$2→6) on their surface. The B16 cell line expresses mainly GM3 but hardly expresses other gangliosides (references (13), (15), (49) - (51)). The cells were incubated with the phage clones as well as the bound clones and labeled with fluoresceine-conjugate antibody. The fluorescence in the cells was measured by a flow cytometer and observed fluorescence intensity was plotted as a function of phage concentration (Fig. 3).

The B16 cells were incubated at 0°C for 1 hour in 1% BSA/PBS with the c01-phage clone (black circles), c03-phage clone (white circles), or the control phage clone (the wild type; black triangles). The bound phage clones were labeled with a primary anti-phage antibody and a secondary FITC-conjugate antibody. The amount of bound phage clones to the cells was determined by a flow cytometer.

The c01-phage clone was bound to B16 cells at phage concentrations ranging from 1 to 10 nM. In contrast, no significant binding was observed for c03 or the control phage clone.

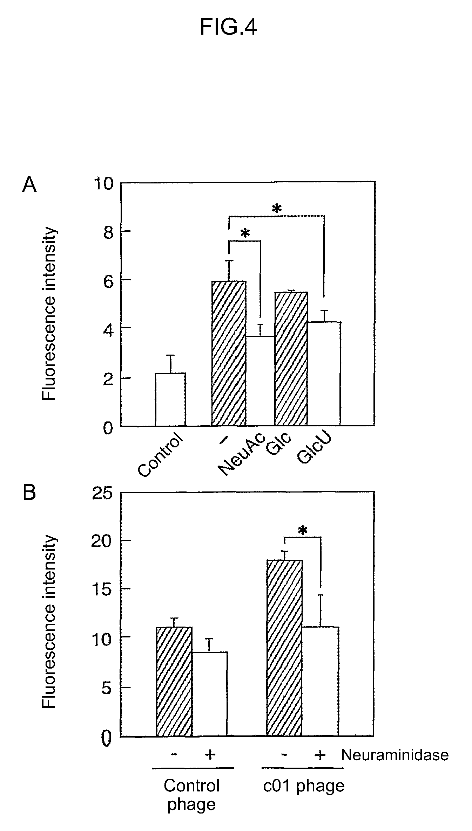

To ascertain whether the binding of the phages is mediated by sialic acid on the cell surface, an inhibition assay and neuraminidase treatment of the cells were performed. The binding of the c01-phage clone (10 nM) to B16 cells was inhibited by the inversion of 1 mM NeuAc (Fig. 4A).

FIG. 4A shows the inhibition of the binding between the c01-phage clone and B16 cells by a monosaccharide. B16 cells (2 x 10^5 cells) were incubated with phage clones (10 nM) in the absence (-) or presence of NeuAc, Glc, or GlcU (1 mM).

A slight inhibition was observed in the presence of glucuronic acid (GlcU). Since GlcU is, like NeuAc, an acidic sugar, carboxylic acid is suggested to be partially responsible for the interaction between phages and the sugar chain. In contrast, no inhibition was observed by the addition of glucose. Neuraminidase (sialidase) from Arthrobacter ureafaciens preferentially hydrolyzes terminal $\alpha2\rightarrow3$ and $\alpha2\rightarrow6$-bonded sialic acid of a glycoconjugate (relative activity: $\alpha2\rightarrow6$ > $\alpha2\rightarrow3$ > $\alpha2\rightarrow8$), reference (52)). B16 cells were treated with neuraminidase in a buffer (pH 6.5) at 37°C for 1.5 hours. Under this condition, the amount of the sialic acid removed from the cells was inferred to be 13 $\mu$g for 10^6 cells, which is almost equal to the total amount of NeuAc in the existing glycoprotein (references (49) - (50)). On the other hand, decomposition of NeuAc from GM3 was not observed by a thin-layer chromatography analysis (data not shown). Sialic acid in the purified GM3 was digested by neuraminidase. However, treatment of the whole cells did not show dissociation of sialic acid from GM3. It was therefore considered that when cells were treated with neuraminidase the sialyl glycoconjugate on the cells was only GM3. Although ELISA showed that the c01-phage clone binds to GM3, the binding of c01-phages to neuraminidase-treated B16 cells was decreased to the same level as the control phage (FIG. 4B).

FIG. 4B shows the effect of the neuraminidase treatment on the phage binding to B16 cells. B16 cells (2 x 10^5 cells) were incubated with 0.05 unit of neuraminidase (pH 6.5) in 1% BSA/PBS at 37°C for 90 min, followed by further incubation with the phage clones. In FIG. 4B, the asterisk denotes $p < 0.05$.

When cells were treated with neuraminidase at pH 7.4, which is the conditions where the enzyme is inactive, the c01-phages were bound to the cells (data not shown). These results indicate that the c01-phage clone binds to sialyloligosaccharide on glycoprotein, not to GM3. It is possible that this affinity is due to a steric hindrance by the phage, because a phage is a flexible filament (about 1 $\mu$m in length, 6 to 10 nm in thickness) (reference (26)). Based on the results of ELISA (FIG. 2), it is inferred that the c01-phage clone interacts with the $\alpha2\rightarrow6$-bonded sialylgalactose in glycoprotein.

(e) Sugar recognition by synthetic 15-mer peptides

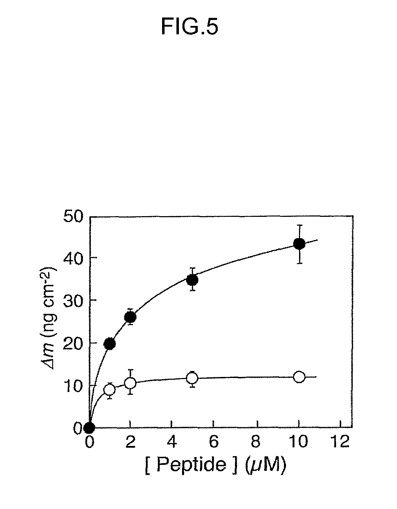

To investigate sugar recognition of the selected peptides, the 15-mer peptides H-GWWYKGRARPVSAVA-NH$_2$ (c01-peptide; SEQ ID NO. 1) and H-RAVWRHSVATPSHSV-NH$_2$ (c03-peptide; SEQ ID NO. 2) were synthesized. The binding assay of these peptides to a glycolipid was analyzed by the 27-MHz QCM method (the sensitivity of the 27-MHz QCM is about 10-fold higher than that of a 9-MHz QCM) (references (44) - (46)). A monolayer of glycolipid (GM3, 6' GM3, LacCer, GalCer, or GlcCer) was prepared at the air-water interface and transferred to the QCM gold surface. Then, the peptide solution was injected into the QCM cuvette and the amount of peptide binding was monitored as a function of time (data not shown). The binding of the peptide to the monolayer ($\Delta$m) was plotted as a function of peptide concentration, as shown in FIG. 5.

The values of mass increase in response to the c01-peptide (black circle) and the c03-peptide (white circle) bound at equilibrium ($\Delta$m, ng cm$^{-2}$) were in the range of 1.0 to 10 $\mu$M as the final peptide concentration by the QCM analysis. The maximum binding amount ($\Delta$m$_{max}$) and the dissociation constant (K$_d$) were calculated from the mutual plot by using equation II (Table 2) (references (44)-(45), (47)). The K$_d$ value is equivalent to the peptide concentration required for 50% of the maximum bindings. The c01-peptide and the c03-peptide had the highest affinity to GM3 at the K$_d$ value of 1.8 and 0.34 $\mu$M, respectively. The maximum binding amount of the c01-peptide (46 ng cm$^{-2}$) was larger than that of the c03-peptide (11 ng cm$^{-2}$). However, although these peptides also had affinity to 6'GM3 and LacCer, the K$_d$ values was higher than the K$_d$ value for GM3. These two peptides either had a low binding to GalCer and GlcCer or were bound to neither of them. Accordingly, these peptide seemed to have interacted with the terminal NeuAc-Gal structure. Meanwhile, the control peptide (H-AETVESCLAKPHTEN-NH$_2$) was bound to none of the glycosphingolipids used in this study (data not shown).

Table 2

| | | c01-peptide | | c03-peptide | |
|---|---|---|---|---|---|
| Glyco-lipid | Structure[a] | $\Delta$m$_{max}$[b] (ng cm$^{-2}$) | K$_d$[b] ($\mu$M) | $\Delta$m$_{max}$ (ng cm$^{-2}$) | K$_d$ ($\mu$M) |
| GM3 | NeuAc($\alpha$2-3)GalGlcCer | 46±2.4 | 1.8±0.4 | 11±0.1 | 0.34±0.005 |
| 6'GM3 | NeuAc($\alpha$2-G)GalGlcCer | 93±4.6 | 4.0±1.1 | 56±3.1 | 7.0±2.2 |

(continued)

| | | Synthetic peptides bound to ganglioside and glycosphingolipid | | | |
| | | c01-peptide | | c03-peptide | |
| Glyco-lipid | Structure[a] | $\Delta m_{max}$[b] (ng cm$^{-2}$) | $K_d$[b] ($\mu$M) | $\Delta m_{max}$ (ng cm$^{-2}$) | $K_d$ ($\mu$M) |
|---|---|---|---|---|---|
| LacCer | <u>Gal</u>GlcCer | 74±5.2 | 4.8±1.6 | 37±1.8 | 5.9±1.2 |
| GalCer | <u>Gal</u>Cer | 64±4.5 | 13 ±3.3 | <3 | - |
| GleCer | GlcCer | n.d.[c] | - | n.d. | - |

a. The region recognized by the peptide is underlined.
b. The $\Delta m_{max}$ and $K_d$ values were obtained by QCM analysis (1 Hz = 0.91 ng cm$^{-2}$).
c. Not detected.

As shown in Example 1, the method of phage library selection was used for determination of glycolipid-binding peptide sequences in combination with the lipid monolayer at an air-water interface (reference (35)). In this method, since glycolipid molecules are highly oriented at a surface pressure of 30 mN m$^{-1}$ (0.4nm$^2$molecule$^{-1}$), only sugar chains are exposed to the water phase. As a result, phage molecules can interact only with sugar chains. In this study, GM3 was used as target molecules for the affinity selection because GM3 has sialylgalactose residues, the main oligosaccharides present on the cell surface. The GM3-binding phages were enriched by four rounds of affinity selection. The enrichment was detected by the quartz-crystal microbalance at each round (data not shown). Finally, an about 6.5-fold mass increase was detected (FIG. 1). The QCM frequency decrease (26 Hz) observed at the fourth round corresponds to the amount of the saturation binding of the phages on the QCM electrode.

Seven kinds of peptide sequences were deduced from the DNA sequences of the 27 isolated phage clones. The seven peptide sequences were classified into two consensus motifs, W-xxxA-R and WRx-VxFxS (Table 1). The arginine and the aromatic amino acids in the consensus motifs were also observed in GM1-binding peptides (reference (35)). Further, the sequence of the cD1-peptide was the same as that of GM1-binding peptide (GWWYKGRARPVSAVA). 15-mer c01- and c03-peptides containing the selected sequences were chemosynthesized. Their binding affinities to several glycolipids were examined and the sugar portion involved in the interaction with the peptides was determined (FIG. 5 and Table 2). The fact that the two peptides have the minimum $K_d$ values to GM3 suggested that the terminal NeuAc-Gal structure is required for carbohydrate recognition by the peptide. It was therefore inferred that (R), Trp (W), Ala (A), Val(V), Phe (F) and Ser (S) in the peptide motifs were involved in the interaction with NeuAc-Gal of GM3. A hydroxyl group of a sugar serves simultaneously as a hydrogen bond donor and acceptor (references (53), (54)). In many cases, an amide group of a peptide further serves as a hydrogen bond donor and a carbonyl group or a carboxylic acid group of a peptide and NeuAc serve as hydrogen bond acceptors. In addition, a B side of a galactopyranose ring and a methyl group of NeuAc can interact with the aromatic rings of Trp and Phe. The consensus motifs of GM3-binding peptides were composed of sugar-binding amino acids. Therefore, these peptides can recognize the NeuAc-Gal portion by hydrogen bonding and van der Waals interactions.

The sialylgalactose (NeuAc-Gal) structure is the terminal sugar sequence in glycoproteins and glycolipids. Since mouse B16 melanoma cells mainly express GM3 but hardly express other gangliosides, the B16 cells were used for determining the affinity of selected phages for GM3 on the cell surface (references (13), (15), (49)-(50)). When B16 cells were incubated with the selected phage clones, only the c01-phage clone was bound to the cells (FIG. 3). The removal of sialic acids from the cells and the addition of free N-acetyl neuraminic acids resulted in the inhibition of phage binding to the cells. These results suggested that the binding of the c01-phage clone is mediated by the sialic acid residue of a glycoprotein. The c01-phage clone had affinities to both Neu$\alpha$2→3Gal and Neu$\alpha$2→6Gal bonds, and the c03-phage clone had an affinity to the Neu$\alpha$2→3Gal bond (FIG. 2). This indicated that only the phage clone c01 interacts with Neu$\alpha$2→6Gal on the surface of the B16 cells. Although GM3 is expressed on the surface of B16 cells, phage binding to the cells was not observed. Neuraminidase was not able to digest GM3-NeuAc. These results revealed that large molecules (such as a phage clone and an enzyme) are incapable of approaching GM3 on a B16 cell.

EXAMPLE 2

Experiment on suppression of influenza virus infection Plaque assay

Inhibition of influenza virus infection was determined by a plaque assay for MDCK cells. The MDCK cells in 6-well plates are incubated with 0.2 mL of influenza virus A/PR/8/34 solution (100 to 200 pfu; pfu denotes plaque-forming units) containing the liposome or the peptide-containing liposome. After a 30 min incubation at 37°C under 5% CO$_2$, supernatant was removed and the cells were washed with PBS. 2 mL (per well) of 2 x MEM + BSA containing 0.6% agarose, 0.01% of O-diethylaminoethyl cellulose dextran, 0.1% $NaHCO_3$, and 0.01 $\mu$/m acetyl trypsin was added and the cells were incubated for two days. Viable cells were stained with crystal violet solution (1 mg/mL in 20% ethanol) and the number of plaques was counted. The maximum infection activity (100%) was defined as the number of plaques in the case of the absence of a liposome. The $IC_{50}$ value (50% inhibitory concentration) of the peptide-containing liposome was obtained from the plot between log [f/(1-f)] and log [peptide-containing liposome], where f is the percent infection activity.

The results of the examination of inhibition of influenza virus infection into MDCK cells by a peptide-containing liposome were as in the following.

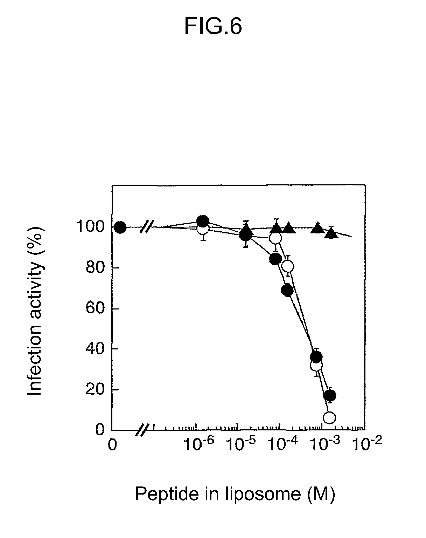

Inhibition of influenza virus infection into MDCK cells by the peptide-containing liposomes was determined by a plaque assay. The peptide was acylated at the N-termirus by using a stearoyl group and incorporated into a PC/cholesterol liposome (peptide-containing liposome). Since both the synthetic c01 and c03-peptides had affinities to the NeuAc$\alpha$2→3Gal and NeuAc$\alpha$2→ 6Gal structures, the peptide-containing liposome is capable of binding to a sialyloligosaccharide on the MDCK cell. In the presence of the peptide-containing liposome, the infection by the influenza virus A/PR/8/34 (H1N1) into the MDCK cells was inhibited (FIG. 6).

MDCK cells were incubated for 30 min with influenza virus A/PR/8/34 in the presence of a c01-peptide-containing liposome (black circles), a c03-peptide-containing liposome (white circles), or a liposome alone (black triangles). The cells were washed and incubated for two days. Then, viable cells were stained and the number of plaques was counted.

The $IC_{50}$ values of the c01-peptide-containing liposome and the c03-peptide-containing liposome were 0.36 and 0.37 mM, respectively (Table 3). The control peptide sequence did not exhibit inhibition. The c01 and c03-phage clones did not have an affinity to the influenza virus (data not shown). Therefore, it was shown that the binding of these liposomes to the surface of MDCK cells inhibits influenza virus infection.

Table 3

| Inhibition of the influenza type A virus infection by N-stearoyl peptide in liposomes | | | |
|---|---|---|---|
| Liposome | PC/cholesterol/peptide (Mol:mol:mol) | N-Stearoyl peptide | $IC_{50}$ (mM) |
| c01-Peptide/liposome | 20:10:3 | $C_{17}H_{35}CO$-GWWYKGRARPVSAVA-$NH_2$ | 0.36 |
| C03-Peptide/liposome | 20:10:3 | $C_{17}H_{35}CO$-RAVWRHSVATPSHSV-$NH_2$ | 0.37 |
| pIII Peptide/liposome | 20:10:3 | $C_{17}H_{35}CO$-AETVESCLAKPHTEN-$NH_2$ | >10 |
| Liposome | 20:10:0 | - | >10 |

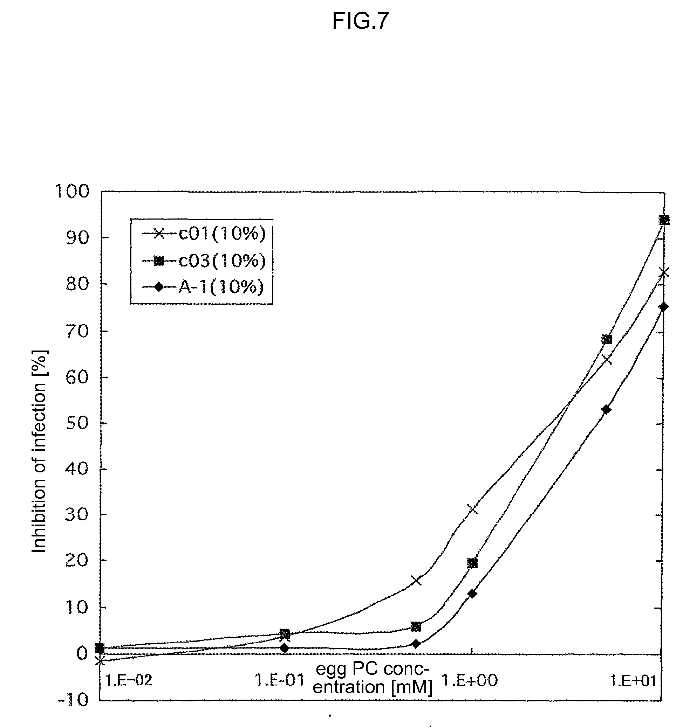

In addition, a similar experiment was carried out with liposomes containing the c01-peptide and the c03-peptide, both of which are ganglioside GM3-binding peptides, and the influenza hemagglutinin-binding peptide A-1 (ARLSPTMVHPRGAQP (SEQ ID NO. 8)). The results are shown in FIG. 7, in which the horizontal axis indicates the concentrations (mM) of egg PC in the liposomes and the vertical axis indicates percent inhibition of infection (%). The percent inhibition of infection is calculated as a ratio of decrease in the number of plaques when the liposome containing the peptide is added compared to the number of plaques when the liposome not containing a peptide is added. As shown in FIG. 7, all the three peptides inhibited influenza virus infection, among which the ganglioside GM3-binding peptides showed a higher inhibition: the percent inhibition of infection at an egg PC concentration of 1 mM (peptide concentration: 0.15 mM) was 83%, 94%, and 76% for the c01-peptide, the c03-peptide, and the A1 peptide, respectively.

Many kinds of toxins and viruses recognize sugar chain on the cell surface in the early step of infection (reference (6)). It is considered that amyloid $\beta$ protein also interacts with ganglioside GM1 and thus the amyloid - GM1 complex leads to fibril formation (references (55), (56)). Several compounds that inhibit the interaction between pathogenic molecules and sugar chains are under development for medical application (references (57), (58)). In the present invention, synthetic peptides were introduced into liposomes to evaluate their functions as influenza virus suppressors. The N-terminus of the peptides were acylated with a stearoyl group to provide a hydrophobic portion. As had been expected, the peptide-containing liposomes inhibited influenza virus (H1N1 type) infection into MDCK cells (FIG. 6). Hemagglutinin serotype H1 of influenza type A virus A/PR/8/34 (H1N1) is specific to NeuAc$\alpha$→3Gal bond. A MDCK cell has both $\alpha$2→3 and $\alpha$2→6-bonded sialic acids on their surface (reference (59)). The peptide liposomes were bound to the sialylgalactose portion on MDCK cells, thereby inhibiting the binding of the influenza virus to sialylgalactose receptors on the MDCK cells. Takikawa et al. have recently reported that liposomes containing glyco-replica peptides were bound to cancer cells, thereby inhibiting the metastasis of the cancer cells (reference (58)). The introduction of functional peptides into liposomes can be an effective method for inhibiting the cell-cell and cell-virus interactions.

15-mer peptide sequences which can recognize the sialylgalactose portion were obtained by a phage library selection. The resulting peptides had not only an affinity to the sugar chains of the glycolipid monolayer but also an affinity to the glycoprotein on the cell surface. The binding of the peptides to the cell surface significantly inhibited influenza virus infection. The peptides selected by the present invention can be used as influenza virus suppressors.

A list of references referred to in the present invention are provided below. When a reference in the following list was referred to in the present specification, its reference number was indicated.

Reference list

1. Varki, A. (1993) Glycobiology 3, 97-130

2. Dwek, R. A. (1996) Chem. Rev. 96, 683-720

3. Weis, W. I., and Drickamer, K. (1996) Annu. Rev. Biochem. 65, 441-473

4. Lis, H., and Sharon, N. (1998) Chem. Rev. 98, 637-674

5. Phillips, M. L., Nudelman, E., Gaeta, F. C. A., Perez, M., Singnhal, A. K. , Hakomori, S. -I., and Paulson, J. C. (1990) Science, 250, 1130-1132

6. Karlsson, K.-A. (1989) Annu. Rev. Biochem. 58, 309-350

7. Kolter, T., and Sandhoff, K. (1999) Angew. Chem. Int. Ed. 38, 1532-1568

8. Hakomori, S. , Yamamura, S. , and Handa, A. K. (1998) Ann. N. Y. Acad. Sci. 845, 1-10

9. Thompson, T. E., and Tillack, T. W. (1985) Ann. Rev. Biophys. Biophys. Chem. 14, 361-386

10. Anderson, R. G. W. (1998) Annu. Rev. Biochem. 67, 199-225 11. Hakomori, S.-I. (2000) Glycoconj. J. 17, 143-151

12. Galbiati, F., Razani, B. , and Lisanti, M. P. (2001) Cell 106, 403-411

13. Iwabuchi, K. , Zhang, Y., Handa, K., Withers, D. A., Sinay, P., and Hakomori, S. (2000) J. Biol. Chem. 275, 15174-17181

14. Prinetti, A., Iwabuchi, K., and Hakomori, S. (1999) J. Biol. Chem. 274, 20916-20924

15. Iwabuchi, K. , Yamamura, S. , Prinetti, A. , Handa, K. , and Hakomori, S. (1998) J. Biol. Chem. 273, 9130-9138

16. Yuan, C., and Johnston, L. J. (2000) Biophys. J. 79, 2768-2781 17. Mou, J. , Yang, J. , and Shao, Z. (1995) J. Mol. Biol. 248, 507-512

18. Vie, V., Mau, N. V., Lesniewska, E., Goudonnet, J. P., Heitz, F., and Le Grimellec, C. (1998) Langmuir 14, 4574-4583

19. Sato, T. (2001) Trends Glycosci. Glycotechnol. 13, 231-238 20. Hashizume, M., Sato, T., and Okahata, Y. (1998) Chem. Lett. 399-400

21. Sato, T. , Serizawa, T., Ohtake, F. , Nakamura, M., Terabayashi, T. , Kawanishi, Y. , and Okahata, Y. (1998) Biochim. Biophys. Acta 1380, 82-92

22. Sato, T., Serizawa, T., and Okahata, Y. (1996) Biochim. Biophys. Acta 1285, 14-20

23. Sears, P., and Wong, C. -H. (1999) Angew. Chem. Int. Ed. 38, 2300-2324

24. Feizi, T. (1985) Science 314, 53-57

25. Scott, J. K., and Smith, G. P. (1990) Science 249, 386-390 26. Smith, G. P., and Scott, J. K. (1993) Methods Enzymol. 217, 228-257

27. Yamamoto, K., Maruyama, I. N. , and Osaka, T. (2000) J. Biochem. (Tokyo) 127, 137-142

28. Wang, L. , Radic, M. Z., Siegel, D. , Chang, T. , Bracy, J. , and Galili, U. (1997) Mol. Immunol. 34, 609-618

29. Peletskaya, E. N. , Glinsky, V.V., Glinsky, G. V. , Deutscher, S. L. , and Quinn, T. P. (1997) J. Mol. Biol. 270, 374-384

30. Peletskaya, E. N., Glinsky, G. , Deutscher, S. L. , and Quinn, T. P. (1996) Mol. Divers. 2, 13-18

31. Gui, J. , Moyana, T. , and Xiang, J. (1996) Biochem. Biophys. Res. Commun. 218, 414-419

32. Gui, J. , Moyana, T. , Malcolm, B. , and Xiang, J. (1996) Proteins 24, 352-358

33. Mao, S. , Gao, C. , Lo, C. H. , Wirsching, P. , Wong, C. -H. , and Janda, 1999) Proc. Natl. Acad. Sci. U S A 96, 6953-6958

34. Dinh, Q., Weng, N. P., Kiso, M., Ishida, H., Hasegawa, A., and Marcus, D. M. (1996) J. Immunol. 157, 732-738

35. Matsubara, T., Ishikawa, D., Taki, T., Okahata, Y., and Sato, T. (1999) FEBS Lett. 456, 253-256

36. Qiu, J. X., Kai, M. , Eduardo, A. P., and Marcus, D. M. (1999) J. Neuroimmunology 97, 172-181

37. van Kuppevelt, T. H. , Dennissen, M. A., van Venrooij, W. J. , Hoet, R. M. , and Veerkamp, J. H. (1998) J. Biol. Chem. 273, 12960-12966

38. Burg, M. A., Pasqualini, R. , Arap, W. , Ruoslahti, E. , and Stallcup, W. B. (1999) Cancer Res. 59, 2869-2874

39. Reason, D. C., Wagner, T. C., and Lucas, A. H. (1997) Infect. Immun. 65, 261-266

40. Deng, S. , MacKenzie, C. R. , Sadowska, J. , Michniewicz, J. , Young, N. M., Bundle, D. R., and Narang, S. A. (1994) J. Biol. Chem. 269, 9533-9538

41. Williams, M. N. V., Freshour, G., Darvill, A. G., Albersheim, P. , and Hahn, M. G. (1996) Plant Cell 8, 673-685

42. Hashizume, M. , Sato, T. , and Okahata, Y. , in preparation. 43. Nishi, T. , Budde, R. J. A. , McMurry, J. S. ,

Obeyesekere, N. U. , Safdar, N., Levin, V. A., and Saya, H. (1996) FEBS Lett. 399, 237-240

44. Okahata, Y. , Niikura, K. , Sugiura, Y. , Sawada, M. , and Morii, T. (1998) Biochemistry 37, 5666-5672
45. Niikura, K., Matsuno, H., and Okahata, Y. (1999) Chem. Eur. J. 5, 1609-1616
46. Niikura, K., Matsuno, H., and Okahata, Y. (1998) J. Am. Chem. Soc. 120, 8537-8538
47. Sauerbrey, G. (1959) Z. Phys. 155, 206-222
48. Ebara, Y. , and Okahata, Y. (1994) J. Am. Chem. Soc. 116, 11209-11212
49. Moretti, S., Montorfano, G., Rapelli, S., andBerra, B. (1997) ITAL. J. Bioc. 46, 187-195
50. Schroeder, F., and Gardiner, J. M. (1984) Cancer Res. 44, 3262-326
51. Corffield, A. P., Higa, H., Paulson, J. C., and Schauer, R. (1983) Biochim. Biophys. Acta 744, 121-126
52. Suzuki, Y. , Nagano, Y. , Kato, H. , Matsumoto, M. , Nerome, K. , Nakajima, K. , and Nobusawa, E. (1986) J. Biol. Chem. 261, 17057-17061
53. Weis, H., and Sharon, N. (1998) Chem. Rev. 98, 637-6
54. Quiocho, F. A. (1989) Pure & Appl. Chem. 61, 1293-1306
55. Yanagisawa, K. , Odaka, A. , Suzuki, N., and Ihara, Y. (1995) Nature Med. 1, 1062-1066
56. Kakio, A. , Nishimoto, S., Yanagisawa, K., Kozutsumi, Y., Matsuzaki, K. (2001) J. Biol. Chem. 276, 24985-24990
57. Totani, K., Kubota, T., Kuroda, T., Murata, T., Hidari, K. I. -P. J., Suzuki, T. Suzuki, Y., Kobayashi, K., Ashida, H., Yamamoto, K., and Usui, T. (2003) Glycobiology 13, 315-326
58. Takikawa, M., Kikkawa, H., Asai, T., Yamaguchi, N., Ishikawa, D., Tanaka, M. , Ogino, K. , Taki, T. , and Oku, N. (2000) FEBS Lett. 466, 381-384
59. Stray, S. J. , Cummings, R. D., and Air, G. M. (2000) Glycobiology 10, 649-658

Industrial Applicability

The present invention provides influenza virus infection suppressors which inhibit binding of influenza virus to host cells by blocking receptors on host cells.

The influenza virus infection suppressor according to the present invention blocks a receptor on a host cell. Therefore, even when administered to an uninfected subject, the suppressor can suppress the subsequent influenza virus infection, and thus can be used as an effective infection-preventive agent. Since the influenza virus infection suppressor according to the present invention blocks the cellular receptor recognized by influenza viruses, the suppressor can suppress the infection regardless of the types of the influenza virus, and it can be used to suppress infection not only by the influenza viruses isolated from human, such as type A or type B, but also by influenza viruses isolated from birds, etc. Further, even after an influenza virus infection has been established, the influenza virus infection suppressor according to the present invention can suppress a reinfection by the influenza virus that has been replicated by budding, therefore it can be effectively used as a therapeutic agent for influenza as well. Since the influenza virus infection suppressor according to the present invention contains a peptide having ten and a few amino acids as an active ingredient, it can be easily synthesized and prepared, and thus utilized as a pharmaceutical composition in a wide variety of forms.

All publications cited in the present specification are entirely incorporated herein by reference. One skilled in the art will readily recognize that various changes and modifications can be made therein without departing from the technical ideas and the scope of the invention as disclosed in the following claims. The present invention intends to encompass such changes and modifications.

**Claims**

1. An influenza virus infection suppressor comprising a sialylgalactose-binding peptide as an active ingredient.

**2.** The influenza virus infection suppressor of claim 1, wherein the sialylgalactose-binding peptide is a ganglioside GM3-binding peptide.

**3.** The influenza virus infection suppressor of claim 1, comprising the sialylgalactose-binding peptide of the following

(a) or (b) as an active ingredient:
(a) a peptide having the amino acid sequence of SEQ ID NO. 1 or 2;
(b) a peptide having an amino acid sequence wherein one or several amino acids are deleted, substituted, or added, from/to the amino acid sequence of SEQ ID NO. 1 or 2, and having ganglioside GM3-binding activity.

**4.** The influenza virus infection suppressor of any one of claims 1 to 3, wherein the sialylgalactose-binding peptide is alkylated or lipidized.

**5.** The influenza virus infection suppressor of any one of claims 1 to 3, wherein the sialylgalactose-binding peptide is contained in a liposome.

**6.** The influenza virus infection suppressor of any one of claims 1 to 5, wherein the influenza virus is selected from the group consisting of type A influenza, type B influenza, and type C influenza.

**7.** The influenza virus infection suppressor of any one of claims 1 to 5, wherein the influenza virus is an avian influenza or a swine influenza.

**8.** The influenza virus infection suppressor of any one of claims 1 to 7, further comprising a pharmaceutically acceptable carrier.

**9.** A method for preventing or treating influenza, comprising a step of administering the influenza virus suppressor of any one of claims 1 to 8 to a nonhuman animal.

**10.** The method for preventing or treating influenza of claim 9, wherein the nonhuman animal is a bird or a swine.

FIG.1

Incubation time (min)

FIG.2

Relative bindig affinity

FIG.3

# FIG.4

FIG.5

FIG.6

Peptide in liposome (M)

## FIG.7

## INTERNATIONAL SEARCH REPORT

| | |
|---|---|
| International application No. |
| PCT/JP2004/003031 |

A. CLASSIFICATION OF SUBJECT MATTER
    Int.Cl⁷ A61K38/00, A61P31/16, 43/00

According to International Patent Classification (IPC) or to both national classification and IPC

B. FIELDS SEARCHED

Minimum documentation searched (classification system followed by classification symbols)
    Int.Cl⁷ A61K38/00, A61P31/16, 43/00

Documentation searched other than minimum documentation to the extent that such documents are included in the fields searched

Electronic data base consulted during the international search (name of data base and, where practicable, search terms used)
    CAPLUS(STN), REGISTRY(STN), MEDLINE(STN), BIOSIS(STN),
    PIR/Swissprot/Geneseq

C. DOCUMENTS CONSIDERED TO BE RELEVANT

| Category* | Citation of document, with indication, where appropriate, of the relevant passages | Relevant to claim No. |
|---|---|---|
| A | JP 2002-284798 A (Keio University), 03 October, 2002 (03.10.02), (Family: none) | 1-8 |
| A | JP 2001-233773 A (Toko Yakuhin Kogyo Co., Ltd.), 28 August, 2001 (28.08.01), (Family: none) | 1-8 |
| A | WO 00/59932 A1 (Otsuka Pharmaceutical Co., Ltd.), 12 October, 2000 (12.10.00), & EP 1167382 A1 | 1-8 |

| | | | |
|---|---|---|---|
| ☒ Further documents are listed in the continuation of Box C. | | ☐ See patent family annex. | |

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search     12 May, 2004 (12.05.04) | Date of mailing of the international search report     01 June, 2004 (01.06.04) |
| Name and mailing address of the ISA/     Japanese Patent Office | Authorized officer |
| Facsimile No. | Telephone No. |

Form PCT/ISA/210 (second sheet) (January 2004)

**INTERNATIONAL SEARCH REPORT**

| International application No. |
|---|
| PCT/JP2004/003031 |

C (Continuation). DOCUMENTS CONSIDERED TO BE RELEVANT

| Category* | Citation of document, with indication, where appropriate, of the relevant passages | Relevant to claim No. |
|---|---|---|
| A | WO 97/22615 A1 (Daikin Industries, Ltd.), 26 June, 1997 (26.06.97), & EP 812855 A1 & US 5789561 A | 1-8 |
| A | JP 10-072355 A (Snow Brand Milk Products Co., Ltd.), 17 March, 1998 (17.03.98), (Family: none) | 1-8 |
| A | JP 5-279379 A (Snow Brand Milk Products Co., Ltd.), 26 October, 1993 (26.10.93), (Family: none) | 1-8 |
| A | JP 2000-253900 A (Otsuka Pharmaceutical Co., Ltd.), 13 September, 2000 (13.09.00), (Family: none) | 1-8 |
| A | Matsubara T., Ganglioside-binding peptides selected from a combinatorial library and their carbohydrate recognition, Trends in Glycoscience and Glycotechnology, Vol.13, No.73, 2001, pages 557 to 560 | 1-8 |

Form PCT/ISA/210 (continuation of second sheet) (January 2004)

## INTERNATIONAL SEARCH REPORT

| International application No. |
| --- |
| PCT/JP2004/003031 |

---

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 9, 10
    because they relate to subject matter not required to be searched by this Authority, namely:
    Claims 9 and 10 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**    ☐ The additional search fees were accompanied by the applicant's protest.

☐ No protest accompanied the payment of additional search fees.

Form PCT/ISA/210 (continuation of first sheet (2)) (January 2004)

**INTERNATIONAL SEARCH REPORT**

International application No.

PCT/JP2004/003031

<Subject of search>

Claims 1, 2 and 4 to 8 relate to remedies containing, as the active ingredient, compounds defined by a desired property "a sialylgalactose group-binding peptide" or "a ganglioside GM3-binding peptide". Although claims 1, 2 and 4 to 8 involve any compounds having such properties, it appears that only small parts of the claimed compounds are supported by the description in the meaning within PCT Article 6 and disclosed therein in the meaning within PCT Article 5.

Even though the common technical knowledge at the point of the application is taken into consideration, the scopes of the compounds having the properties "a sialylgalactose group-binding peptide" or "a ganglioside GM3-binding peptide" cannot be specified. Thus, claims 1, 2 and 4 to 8 do not comply with the requirement of clearness under PCT Article 6 too.

Such being the case, the search was made on the relationship between "a sialylgalactose group-binding peptide" and "a ganglioside GM3-binding peptide" and an influenza-infection inhibitor and a remedy comprising the compound specified in claim 3 as the active ingredient. Complete search was made on claim 3.

Form PCT/ISA/210 (extra sheet) (January 2004)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002284798 A
- WO 059932 A
- JP 2000253900 A
- WO 0059932 A

**Non-patent literature cited in the description**

- **SCOTT, J. M. ; SMITH, G. P.** *Science,* 1990, vol. 249, 386-390
- **SMITH, G. P. ; SCOTT, J. K.** *Methods in Enzymology,* 1993, vol. 217, 228-257
- *Biochim. Biophys. Acta,* 1998, vol. 1138, 82-92
- *Biochim. Biophys. Acta,* 1996, vol. 1285, 14-20
- *New Current,* 2000, vol. 11 (3), 15-20
- *Biochemica et Biophysica Acta.,* 1992, vol. 1128, 44-49
- *FEBS Letters,* 1997, vol. 413, 177-180
- *J. Biol. Chem.,* 1982, vol. 257, 286-288
- *Biochim. Biophys. Acta,* 1975, vol. 394, 483-491
- *Biochim. Biophys. Acta,* 1976, vol. 443, 629-634
- **SZOKA et al.** *Proc. Natl. Acad. Sci. U. S. A,* 1978, vol. 75, 4194-4198
- *FEBS lett.,* 1979, vol. 99, 210-214
- *Chem. Pharm. Bull,* 1984, vol. 32, 2442-2445
- *Chem. Pharm. Bull.,* 1985, vol. 33, 2916-2923
- *J. Pharm. Sci.,* 1982, vol. 71, 806-812
- *Biochim. Biophys. Acta,* 1979, vol. 557, 9-23
- *Biochim. Biophys. Acta,* 1980, vol. 601, 559-571
- Shishitsu no Kagaku. Liposome. Tokyo Kagaku Dojin, 1974
- Long Circulating Liposomes: old drug. **WOODLE et al.** New therapeutic. Springer-Verlag, 1998
- **Y. NAMBA ; N. OKU.** Liposomal applications to cancer therapy. *J. Bioact. Compat. Polymers,* 1993, vol. 8, 158-177
- **VARKI, A.** *Glycobiology,* 1993, vol. 3, 97-130
- **DWEK, R. A.** *Chem. Rev.,* 1996, vol. 96, 683-720
- **WEIS, W. I. ; DRICKAMER, K.** *Annu. Rev. Biochem.,* 1996, vol. 65, 441-473
- **LIS, H. ; SHARON, N.** *Chem. Rev.,* 1998, vol. 98, 637-674
- **PHILLIPS, M. L. ; NUDELMAN, E. ; GAETA, F. C. A. ; PEREZ, M. ; SINGNHAL, A. K. ; HAKOMORI, S. -I. ; PAULSON, J. C.** *Science,* 1990, vol. 250, 1130-1132
- **KARLSSON, K.-A.** *Annu. Rev. Biochem.,* 1989, vol. 58, 309-350
- **KOLTER, T. ; SANDHOFF, K.** *Angew. Chem. Int. Ed.,* 1999, vol. 38, 1532-1568
- **HAKOMORI, S. ; YAMAMURA, S. ; HANDA, A. K.** *Ann. N. Y. Acad. Sci.,* 1998, vol. 845, 1-10
- **THOMPSON, T. E. ; TILLACK, T. W.** *Ann. Rev. Biophys. Biophys. Chem.,* 1985, vol. 14, 361-386
- **ANDERSON, R. G. W.** *Annu. Rev. Biochem.,* 1998, vol. 67, 199-225
- **HAKOMORI, S.-I.** *Glycoconj. J.,* 2000, vol. 17, 143-151
- **GALBIATI, F. ; RAZANI, B. ; LISANTI, M. P.** *Cell,* 2001, vol. 106, 403-411
- **IWABUCHI, K. ; ZHANG, Y. ; HANDA, K. ; WITHERS, D. A. ; SINAY, P. ; HAKOMORI, S.** *J. Biol. Chem.,* 2000, vol. 275, 15174-17181
- **PRINETTI, A. ; IWABUCHI, K. ; HAKOMORI, S.** *J. Biol. Chem.,* 1999, vol. 274, 20916-20924
- **IWABUCHI, K. ; YAMAMURA, S. ; PRINETTI, A. ; HANDA, K. ; HAKOMORI, S.** *J. Biol. Chem.,* 1998, vol. 273, 9130-9138
- **YUAN, C. ; JOHNSTON, L. J.** *Biophys. J.,* 2000, vol. 79, 2768-2781
- **MOU, J. ; YANG, J. ; SHAO, Z.** *J. Mol. Biol.,* 1995, vol. 248, 507-512
- **VIE, V. ; MAU, N. V. ; LESNIEWSKA, E. ; GOUDONNET, J. P. ; HEITZ, F. ; LE GRIMELLEC, C.** *Langmuir,* 1998, vol. 14, 4574-4583
- **SATO, T.** *Trends Glycosci. Glycotechnol.,* 2001, vol. 13, 231-238
- **HASHIZUME, M. ; SATO, T. ; OKAHATA, Y.** *Chem. Lett.,* 1998, 399-400
- **SATO, T. ; SERIZAWA, T. ; OHTAKE, F. ; NAKAMURA, M. ; TERABAYASHI, T. ; KAWANISHI, Y. ; OKAHATA, Y.** *Biochim. Biophys. Acta,* 1998, vol. 1380, 82-92

- **SATO, T. ; SERIZAWA, T. ; OKAHATA, Y.** *Biochim. Biophys. Acta,* 1996, vol. 1285, 14-20
- **SEARS, P. ; WONG, C. -H.** *Angew. Chem. Int. Ed.,* 1999, vol. 38, 2300-2324
- **FEIZI, T.** *Science,* 1985, vol. 314, 53-57
- **SCOTT, J. K. ; SMITH, G. P.** *Science,* 1990, vol. 249, 386-390
- **SMITH, G. P. ; SCOTT, J. K.** *Methods Enzymol.,* 1993, vol. 217, 228-257
- **YAMAMOTO, K. ; MARUYAMA, I. N. ; OSAKA, T.** *J. Biochem.,* 2000, vol. 127, 137-142
- **WANG, L. ; RADIC, M. Z. ; SIEGEL, D. ; CHANG, T. ; BRACY, J. ; GALILI, U.** *Mol. Immunol.,* 1997, vol. 34, 609-618
- **PELETSKAYA, E. N. ; GLINSKY, V.V. ; GLINSKY, G. V. ; DEUTSCHER, S. L. ; QUINN, T. P.** *J. Mol. Biol.,* 1997, vol. 270, 374-384
- **PELETSKAYA, E. N. ; GLINSKY, G. ; DEUTSCHER, S. L. ; QUINN, T. P.** *Mol. Divers.,* 1996, vol. 2, 13-18
- **GUI, J. ; MOYANA, T. ; XIANG, J.** *Biochem. Biophys. Res. Commun.,* 1996, vol. 218, 414-419
- **GUI, J. ; MOYANA, T. ; MALCOLM, B. ; XIANG, J.** *Proteins,* 1996, vol. 24, 352-358
- **MAO, S. ; GAO, C. ; LO, C. H. ; WIRSCHING, P. ; WONG, C. -H. ; JANDA.** *Proc. Natl. Acad. Sci. U S A,* 1999, vol. 96, 6953-6958
- **DINH, Q. ; WENG, N. P. ; KISO, M. ; ISHIDA, H. ; HASEGAWA, A. ; MARCUS, D. M.** *J. Immunol.,* 1996, vol. 157, 732-738
- **MATSUBARA, T. ; ISHIKAWA, D. ; TAKI, T. ; OKAHATA, Y. ; SATO, T.** *FEBS Lett.,* 1999, vol. 456, 253-256
- **QIU, J. X. ; KAI, M. ; EDUARDO, A. P. ; MARCUS, D. M.** *J. Neuroimmunology,* 1999, vol. 97, 172-181
- **VAN KUPPEVELT, T. H. ; DENNISSEN, M. A. ; VAN VENROOIJ, W. J. ; HOET, R. M. ; VEERKAMP, J. H.** *J. Biol. Chem.,* 1998, vol. 273, 12960-12966
- **BURG, M. A. ; PASQUALINI, R. ; ARAP, W. ; RUOSLAHTI, E. ; STALLCUP, W. B.** *Cancer Res.,* 1999, vol. 59, 2869-2874
- **REASON, D. C. ; WAGNER, T. C. ; LUCAS, A. H.** *Infect. Immun.,* 1997, vol. 65, 261-266
- **DENG, S. ; MACKENZIE, C. R. ; SADOWSKA, J. ; MICHNIEWICZ, J. ; YOUNG, N. M. ; BUNDLE, D. R. ; NARANG, S. A.** *J. Biol. Chem.,* 1994, vol. 269, 9533-9538
- **WILLIAMS, M. N. V. ; FRESHOUR, G. ; DARVILL, A. G. ; ALBERSHEIM, P. ; HAHN, M. G.** *Plant Cell,* 1996, vol. 8, 673-685
- **NISHI, T. ; BUDDE, R. J. A. ; MCMURRY, J. S. ; OBEYESEKERE, N. U. ; SAFDAR, N. ; LEVIN, V. A. ; SAYA, H.** *FEBS Lett.,* 1996, vol. 399, 237-240
- **OKAHATA, Y. ; NIIKURA, K. ; SUGIURA, Y. ; SAWADA, M. ; MORII, T.** *Biochemistry,* 1998, vol. 37, 5666-5672
- **NIIKURA, K. ; MATSUNO, H. ; OKAHATA, Y.** *Chem. Eur. J.,* 1999, vol. 5, 1609-1616
- **NIIKURA, K. ; MATSUNO, H. ; OKAHATA, Y.** *J. Am. Chem. Soc.,* 1998, vol. 120, 8537-8538
- **SAUERBREY, G.** *Z. Phys.,* 1959, vol. 155, 206-222
- **EBARA, Y. ; OKAHATA, Y.** *J. Am. Chem. Soc.,* 1994, vol. 116, 11209-11212
- **MORETTI, S. ; MONTORFANO, G. ; RAPELLI, S. ; BERRA, B.** *ITAL. J. Bioc.,* 1997, vol. 46, 187-195
- **SCHROEDER, F. ; GARDINER, J. M.** *Cancer Res.,* 1984, vol. 44, 3262-326
- **CORFFIELD, A. P. ; HIGA, H. ; PAULSON, J. C. ; SCHAUER, R.** *Biochim. Biophys. Acta,* 1983, vol. 744, 121-126
- **SUZUKI, Y. ; NAGANO, Y. ; KATO, H. ; MATSUMOTO, M. ; NEROME, K. ; NAKAJIMA, K. ; NOBUSAWA, E.** *J. Biol. Chem.,* 1986, vol. 261, 17057-17061
- **WEIS, H. ; SHARON, N.** *Chem. Rev.,* 1998, vol. 98, 637-6
- **QUIOCHO, F. A.** *Pure & Appl. Chem.,* 1989, vol. 61, 1293-1306
- **YANAGISAWA, K. ; ODAKA, A. ; SUZUKI, N. ; IHARA, Y.** *Nature Med.,* 1995, vol. 1, 1062-1066
- **KAKIO, A. ; NISHIMOTO, S. ; YANAGISAWA, K. ; KOZUTSUMI, Y. ; MATSUZAKI, K.** *J. Biol. Chem.,* 2001, vol. 276, 24985-24990
- **TOTANI, K. ; KUBOTA, T. ; KURODA, T. ; MURATA, T. ; HIDARI, K. I. -P. J. ; SUZUKI, T. ; SUZUKI, Y. ; KOBAYASHI, K. ; ASHIDA, H. ; YAMAMOTO, K.** *Glycobiology,* 2003, vol. 13, 315-326
- **TAKIKAWA, M. ; KIKKAWA, H. ; ASAI, T. ; YAMAGUCHI, N. ; ISHIKAWA, D. ; TANAKA, M. ; OGINO, K. ; TAKI, T. ; OKU, N.** *FEBS Lett.,* 2000, vol. 466, 381-384
- **STRAY, S. J. ; CUMMINGS, R. D. ; AIR, G. M.** *Glycobiology,* 2000, vol. 10, 649-658